(19) Europäisches Patentamt
European Patent Office
Office européen des brevets

(11) EP 1 870 706 A1

(12) EUROPEAN PATENT APPLICATION

(43) Date of publication:
**26.12.2007 Bulletin 2007/52**

(51) Int Cl.:
***G01N 30/06*** *(2006.01)* ***B01D 15/38*** *(2006.01)*
***B01J 31/16*** *(2006.01)* ***G01N 33/28*** *(2006.01)*

(21) Application number: **07252518.1**

(22) Date of filing: **21.06.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK YU**

(30) Priority: **21.06.2006 GB 0612344**

(71) Applicant: **Oil Plus Limited**
**Newbury, Berkshire, RG14 5TR (GB)**

(72) Inventors:
- **Smith, Desmond**
**Newbury**
**Berkshire RG14 5TR (GB)**
- **Smith, Patrick Colin**
**Newbury**
**Berkshire RG14 5TR (GB)**
- **Ham, Sandra**
**Newbury**
**Berkshire RG14 5TR (GB)**

(74) Representative: **Copsey, Timothy Graham et al**
**Kilburn & Strode,**
**20 Red Lion Street**
**London WC1R 4PJ (GB)**

(54) **Method of screening crude oil for low molecular weight naphthenic acids**

(57) A method for the quantitation of the naphthenic acid content of molecular weight less than 600 daltons in a hydrocarbon composition, comprising (i) separating naphthenic acids from the hydrocarbon composition and applying them to a macroreticular ion-exchange resin. The resin is washed and the naphthenic acid-metal ion complexes are eluted from the resin. The number of naphthenic acid-metal ion complexes present in the eluate from the resin are quantitated.

EP 1 870 706 A1

## Description

The present invention relates to a method of screening crude oil to determine to amount of low molecular weight naphthenic acid (LMWA) present. The method provides a means by which crude oil can be screened to assess quality and also purified to remove such impurities. References to screening crude oil include refinery product streams and desalter waters to evaluate fluid qualities in terms of fouling, corrosion, emulsion tendencies and environmental concerns.

There are an increasing number of new oil reservoirs being discovered and developed in recent years that yield heavier oil types (i.e. oils with API gravities of between 15 to 25°). With sustained higher oil prices such oilfields have become more viable. One common trait for these oils is that they show high total acid values and significant naphthenic acid contents. In refineries these oils are known as High Acid Crude (HAC) feedstocks. The actual Total Acid Number (TAN) values (mg KOH / g oil) of these oils does not correlate at all with their risk of forming naphthenate solids or other soap types during reservoir fluid production in oilfields. These soap materials are an increasingly more common and very severe flow assurance problem being encountered worldwide [1 to 6]. For example, there are rare cases of 35° API oils of 0.10 TAN that can yield high amounts of naphthenate solid precipitates during production [5]. Alternative ways of defining the relative organic acid fraction of crude oils is a real need in the oil industry, for both the upstream and downstream industries.

Within the last five or more years there has been an increasing number of oilfields produced with varying amounts of naphthenic acids [1 to 6], or rather, the understanding in the oil industry has reached a level where people are now confident in publishing papers in this area. In truth, naphthenic acid problems have been experienced for at least fifty years in the oil industry, mainly manifesting themselves as stable emulsions and dirty disposal water problems in oilfields, e.g. in Southeast Asia such problems have been observed since the early 1960s. These problems were associated with the formation of strong surfactant properties of soaps of organic acids [1, 4, 5, 6 & 8 to 11]. It is noted that as existing crude oil reservoirs are becoming depleted, the newer reservoirs being discovered now, are ones that in general are classed as immature reservoirs and are commonly biodegraded oils that have lost many components (e.g. alkanes and waxes) and thus the naphthenic acids are found to occur in increasing concentrations [2, 8, 9].

By definition a naphthenic acid is a monobasic carboxyl group attached to a saturated cycloaliphatic structure. It has been accepted in the oil industry that all organic acids in crude oil are called naphthenic acids. Naphthenic acids in crude oils are mixtures of low to high molecular weight organic acids. Such acids can be very water soluble to oil soluble depending on their molecular weight, on the process temperatures their parent fluids are subjected to, on the salinity and pH of their parent waters and finally on their parent fluid processing pressures [1, 2, 5 & 6].

Naphthenic acid nomenclature was always known in peer groups as a misnomer as it implied unsaturation due to the "enic" in its name. Naphthenic acids were discovered over 100 years ago [12]. The old classification indicates that naphthenic acid is a monobasic carboxyl group attached to a saturated cycloaliphatic structure [12]. It has been quoted that all organic acids in crude oil are called naphthenic acids but this may not be true [12]. This is simplistic, as acetic acid is not recognized as a naphthenic acid. Heavy Californian crude oils are known to yield naphthenic and naphtheno - aromatic acids, for example [13, 14, 15].

Naphthenic acids in crude oils are mixtures of low to high molecular weights species varying from approximately 100 to >1300 molecular weight (MW), as shown by various analyses, including mass spectrometric (MS) and Fourier Transform Ion Cyclotron Resonance (FTICR) analyses [6, 7]. In the water phase, naphthenic acids can cause stable reverse emulsions. In the oil phase with residual water, these acids have the potential to react with a host of minerals, which can neutralize the acids to form insoluble salts.

The main reaction product found in actual oilfields is the calcium naphthenic acid soap, known to the oil industry as calcium naphthenate. Analyses of various actual soaps samples from oilfields around the globe indicates that they are often a mixture of one or more soap (including calcium, magnesium, sodium, potassium, iron, and aluminium), with occluded silica/sand, mineral scales, iron hydroxides/oxides and sulphides, formation clay, mud residues e.g. bentonites, flocculated asphaltenes, paraffin waxes, resins and treating chemicals [5, 6, 8]. These soaps and other occluded particles have been known under real conditions for a long time [16, 17]. These adhere to oil water interfaces and can become finely dispersed within the water itself. The oil industry to date has solved the emulsion problems caused by naphthenate soaps using acidic demulsifiers, called "pad busters" or interface draw-offs to separate slop treatments [2 to 6 & 8]. The reverse emulsions in oils and in dirty oilfield water are being treated with water clarifiers, most of these being the cationic water clarifiers [5, 8].

The naphthenic acids which form soaps and salts cause problems from refinery and oilfield crude inlet stock tanks creating slops, in desalters these appear as tight emulsions, lowering the desalting efficiencies. They can also react via double displacement to form high molecular weight foulants trapping other components in a tight matrix to foul heat exchangers, cause foaming in units, create interface pads, dirty disposal water, carry over of sodium, calcium and other metals to bottom fractions and products. There is a range of other problems associated with naphthenic acids including the initiation of gum and/or polymer formations, poor dewatering in product stock tanks, transfer of metals and scale.

The normal method of evaluating crude oils to the refinery with special reference to refining problems involve a lot of wet chemistry including, asphaltenes content, ash & carbon residue contents, TAN ASTM D664, Distillation yield tests. Service companies can perform the high temperature fouling tests with and without antifoulants. GC-MS is carried out to determine the range of naphthenic acids.

For the oilfield, the low molecular naphthenic acids and/or other carboxylic acids are not routinely determined, as no oilfield method exists for performing this analysis. The LMWAs are important in emulsion and desalting work in upstream production processes. A ranking of degree of emulsion or poor desalting efficiencies for upstream crude oil samples prior to the present invention does not exist at present.

Based on the main refinery laboratory tests, crude oil refining slates can be determined and blended depending on the GC-MS results to minimise refining problems of fouling and corrosion without lowering the economics of the refinery. The TAN test is a standard test and estimates total acids not only organic naphthenic acids. GC-MS resolves the naphthenic acids into hundreds of components and not all components are identified as naphthenic acids. Although the refinery attempted to do this via GC-MS, there are still extensive corrosion, emulsion and fouling problems in refineries which are not totally answered.

Corrosion via all acids, not only naphthenic acids is a major problem in refineries from the preheat furnaces to the overheads on to the high temperature heat exchangers e.g. the crude unit bottoms. Refiners have managed some of these corrosion problems by choice of certain stainless steels or titanium metallurgy and a chemical corrosion programme. There is therefore a need for improvement in understanding the corrosion potentials of crude oils.

Fouling of processes, occurs due to the inherent and potential - kinetic cleanliness of the crude, the potential instability of the free radicals produced in heaters to form gums and polymers, reactions of naphthenic acids at elevated temperatures with metals in the entrained water, mainly calcium and iron. The inherent fouling potential of the crude oil implicates several materials which can be included in the sales crude: particulate matter, clay, drilling & completion muds, bentonites, sand, metals as oxides and sulphides, inorganic and organic scales.

The organic scale is mainly the naphthenate soap type, although other scale types have been identified, e.g. phosphorus and nitrogen containing types. Naphthenic acid is found in most crude oils at wide ranging values from ppm to percentage levels. Naphthenic acids influence most of the common problems associated with fouling, corrosion and emulsions in refining crude oils with TAN values from 0.5 upwards.

Naphthenic acids form the natural surfactant in crude oils and trap water. Reactions with alkali and alkaline earth metals give the well-known oil soluble/dispersible salt and soaps we recognize as present in crude oils. These compounds are difficult to remove as removal of calcium, sodium or potassium from soap in a desalter requires breakage of the soap C-O-Metal bond. This is not possible unless the desalting chemical contains an acid, chosen from a mineral acid, phosphorus acids, acetic or sulphonic acid or combinations thereof.

Inefficient removal of the metals means that some of the surfactant soap and a portion of LMWAs are transferred to the water phase as a reverse emulsion requiring water clarifiers. In addition to the transfer of soaps to the water in the desalter, some soaps reside in the crude oil leaving the desalter to the preheat furnaces, preheat exchangers (cause fouling and corrosion) and then to the crude distillation unit. Free LMWA naphthenic acids are very oil soluble at desalter temperatures whilst the HMWAs are present as particulate micelles finely dispersed and hydrogen bonded to water, ensuring their transport to the tower.

These equipment and units will be exposed to fouling as naphthenate soaps and naphthenic acids act as binders for the contaminants. A portion of the free Naphthenic acids can react in these units and equipment to form soaps of the metals mentioned above. There are always free naphthenic acids remaining at the inlet of the crude unit to give a TAN value. The low molecular weight acids are distillable, fractionate into the main fractions and can appear as emulsions in recycle drums, condensate knockout vessels and in the overhead vapours.

Some naphthenic acids (LMW & HMW) in the crude unit at the high inlet temperatures, fracture to lower molecular weight naphthenic acids, a portion of which appear as volatile organic acids, giving a degree of unsaturation in bond breakage to the hydrocarbon fragments of this reaction. This unsaturation is the precursor to gum and olefin polymerization reactions.

Naphthenic acids and naphthenate soaps are natural surfactants, which can cause foaming in the crude units or other units. These can then be physically transported upwards into lower boiling fractions to impact the cleanliness of the product streams. Naphthenate soaps also transport the metal calcium, iron, sodium etc upwards to the trays depositing scales in the units. These metals are found in the diesel and fuel oil streams, which affect their compliance with specifications.

These acids by means of their surfactant properties and oxygen/hydroxyl groups in their molecules, are capable of trapping very fine water droplets via Hydrogen bonding and fine micelle, emulsion formation to cause some product streams to become hazy in stock tanks. The entrained water -surfactant in organic fuel fractions cause pumpability problems in the WSIM test for fuels. The products will be off specifications and require dehazing chemicals to settle and separate off the water or rerun for drying via diatomaceous earth or resins.

Thus Naphthenic acids and soaps affect fouling, corrosion, emulsions, desalting, product quality among the

major negative influences to be experienced in the refineries. Low TANs, light crude oil supplies are becoming depleted and thus there is a high proportion of Naphthenic acid crude oils on world markets. A noticeable change is recorded in the quality of new reservoirs being discovered with respect to these types of naphthenic acid crude oils.

These new available crude oils with high TANs are sold at a discount on world markets ranging from US$3-US$30 a barrel depending on prevailing market values of crude oils, acid values, API gravities and yield values. Thus there is a need to determine the qualities of these crude oils in a refining environment to take advantage of a less expensive crude oil.

The design of typical production processes and the decreases in fluid pressures from wells to separation trains affect the bicarbonate, carbon dioxide and pH equilibrium in produced waters. This provides a driving force for the formation of calcium naphthenate soaps [5, 6, 18, 19]. These calcium naphthenate soaps (including contaminants), can deposit across the oil process system from wellheads, subsea flowlines, separators, chemelectrics, heat exchanger tubes to oil filters and even within oil storage tanks. These deposits can congeal to hardened cement-like structures requiring frequent system shutdowns and physical clean-up periods costing millions of dollars in maintenance costs and lost production [5].

Calcium naphthenate dispersed in crude oils to refineries can sludge out in terminal tanks and cause fouling in lines, desalters and heat exchanger units. In refineries most analyses of the naphthenic acid fractions are important due to implications in the corrosion process [9, 17, 20, 21]. Results are reported on the medium molecular weight (250 - 450 MW) naphthenic acids to enable prediction of overheads corrosion/neutralizer rates [22] and the amount of caustic and/or ammonia double decomposition required to combat naphthenic acids and soaps prior to desalters and distillation units. There is no distinction between high and low molecular weight naphthenic acids in refineries. Prior to the ARN acid discovery [7], mass spectrometric analyses were concentrating in the low molecular weight acids (LMWA) area of up to 320 MW [18].

In the water phase, naphthenic acids and their soaps have been known to cause stable reverse emulsions (i.e. oil droplets in a continuous water phase) in some production systems [5]. In the oil phase with residual water, these acids have the potential to react with a host of minerals, which are capable of neutralizing the acids. The main reaction product found in practice is the calcium naphthenic acid soap (the calcium salt of naphthenic acids). These often become insoluble salts and form solids material that can plug production systems, eventually causing system shutdowns [1 to 6]. Analysis of these collected soap solids has proven that calcium soap is often a major component. Soap agglomerations in wells and separation trains can generate a mixture of magnesium, sodium, potassium, iron, and aluminium soaps with occluded formation-derived sand, silt and clays, mineral scales, iron scales, precipitated asphaltenes, resins, petroleum waxes, mud components and treatment chemicals [5,6].

Several gas chromatography and combined expensive mass spectrometric analytical procedures have not so far been able to give acceptably accurate quantitative determinations of the precursor problematic acids contents, in oils prone to generating these soaps. Other methods involve use of a pilot plant to determine the organic scale-soap probability under a combination of synthesised process conditions, typically using significant volumes of aged 'stabilised' (degassed) crude oil samples [6].

A further development is the identification of a newly defined set of naphthenic acids, called the ARN acids, typically of mass over charge, m/z, of 1230 amu (atomic mass units, or Daltons), which have been identified as being present in significant quantity in many oilfield naphthenate deposits and crude oils [5, 6, 7]. It has been proposed that the specific acid types are one of the main precursor acids present in crude oils that must be available to form the calcium and other naphthenate soap solids [6, 7]. The ARN acid structures have yet to be fully defined [7] but they are important acids and they do form a proportion of the acids isolated by the methodology described in this patent.

Previous and current techniques for analysis of naphthenic acids do not examine the relative amount of high molecular weight naphthenic acids. Expensive techniques are employed which are not valuable on a routine basis or practical in field conditions. Chromatographic techniques, including GC-MS [23], MS [15], resolve the naphthenic acids into hundreds of peaks. Analysis of the naphthenate soaps by a Norwegian research group found a strange high molecular weight acid species, designated ARN acids, 1230+ MW, m/z, z=1, which is tetraprotic and also gives a 615 m-2H/z (z=2) signal [6, 7]. These ARN acids were qualitatively detected as the major components in various naphthenate deposits. Previous researchers emphasised the generalised 250-450 MW areas for naphthenic acids [18, 22]. Thus the ARN acid discovery was a revelation in the naphthenate soap story.

Many laboratory investigations have been performed on naphthenic acids and naphthenate solid soaps within the last five years. There are publications from Total [1, 2, 10], Conoco Phillips and Statoil [7], ExxonMobil [22], Heriot Watt University with Oil Plus [6] and university research students Brandl O. and Havre T.E. [19, 24] of NTNU, Professor J. Sjoblom. Experimental work ranged from normal reaction of naphthenic acids with brine to form soaps [1] performed by Rousseau et al, emulsion studies looking at the effects of naphthenic acids [18], naphthenic acids on silica gel (25), Near Infra red work [26] and LC-MS analysis, after anion exchange, the Acid IER Method (10), to name a few. None of these have correlated a ranking table or prediction of good oils versus bad oils with respect to their potential to form naphthenate soaps. The ARN acids discovery [7] is the building block to further work and presents the opportunity to

use this, in a more realistic methodology, for problematic naphthenate soap predictions.

When evaluating literature search results for most published approaches to naphthenic acids quantification or isolation, it can be seen that there is a practical problem to be solved with respect to the significance of the amount of the naphthenate precursor ARN-type acids present in oils, that would lead to actual naphthenate soap deposition problems within oilfield production systems. One major question is how much of these cyclic acids would need to be present in a crude oil for that oil to be designated a problematic naphthenate-forming oil.

The very instrumental reliant liquid chromatography-mass spectrometry (LC-MS), electrospray mass spectrometry (ES-MS) and gas chromatography-mass spectrometry (GC-MS) techniques employed thus far (start of 2006), are not ideal for routine on-site work in oilfield laboratories [6]. There are no published results on the analytical ranking of the special properties of crude oils relative to known oils with problematic naphthenate soap formation tendency and to oils with no naphthenate soap formation tendency.

There is therefore a need for a process that overcomes the problems inherent in the methods used to date to identify low molecular weight species of naphthenic acids.

According to a first aspect of the invention, there is provided a method for the quantitation of the naphthenic acid content of molecular weight less than 600 daltons in a hydrocarbon composition, comprising

(i) separating naphthenic acids of molecular weight less than 600 daltons from the hydrocarbon composition;
(ii) applying the separated naphthenic acids of molecular weight of less than 600 daltons to a macroreticular ion-exchange resin additionally comprising transition metal ions co-ordinated with an amine or ammonia in which the metal ion amine/ammonia co-ordinated complexes are bound to the surface of the resin;
(iii) washing the resin;
(iv) eluting the naphthenic acid-metal ion complexes from the resin; and
(v) quantitating the number of naphthenic acid-metal ion complexes present in the eluate from the resin.

The present invention therefore provides new methods in analysing the crude oils and product streams in an oilfield and/or a refinery. The methods involve an innovative technique of separation of high and low molecular weight ranges of naphthenic acids. The high molecular weight (600-1300MW) naphthenic acids have been found to be the major acid components of soaps in the foulant materials. The low molecular weight (LMW) naphthenic acids < 600 MW, are recognized to cause emulsion and corrosion problems in crude units, whilst the high molecular weight non distillable naphthenic acids (HMWAs) and HMW soaps in the residual crude bottoms cause fouling and severe corrosion in bottoms heat exchangers. Methods for screening a hydrocarbon containing sample for high molecular weight naphthenic acids are described in co-pending application no. GB 0520847.5.

This technique holds great potential as a screening tool for oil and refinery fractions, for new fields, refinery crude oil slates and product streams. The method can be used on current refinery and production operations for troubleshooting present problems.

In its broadest sense, the naphthenic acid content of a hydrocarbon composition is a reflection of the content of all of the organic acids present in the sample, i.e. carboxylic acids. The term naphthenic acid may also be used more narrowly to describe certain specific organic acid components of crude oil as follows.

Single and multiple fused cyclopentane and cyclohexane rings where the carboxylic acid group is attached to an aliphatic side chain or to a cycloaliphatic ring may be described a naphthenic acids

The American Petroleum Institute (API) definition for naphthenic acids includes structures with single and multiple fused cyclopentane and cyclohexane rings as illustrated in Fig. 1, where the carboxyl group may be attached either to the aliphatic side chain (preferably) or to the cycloaliphatic ring (API 2003).

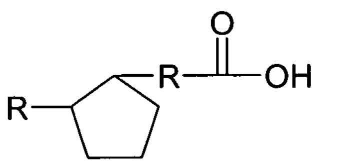

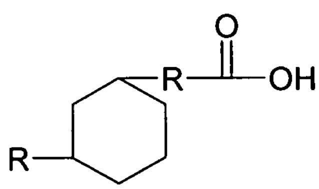

Based on these assumptions, it is possible to represent naphthenic acids by the formula, $C_nH_{2n}+_ZO_2$, where, n is the carbon number and Z represents a homologous series. Z can be 0 or a negative even integer (-2, -4, etc) and reflects the hydrogen loss or deficiency which occurs when a ring is included in the acid structure. Thus, Z = -2 equals one cycloaliphatic ring, Z = -4 equals 2 cycloaliphatic rings, etc. More than one isomer will typically exist for a given Z group. R may be any alkyl, or alkenyl group which may be linear or branched chain, or a ring or connected system of rings, optionally substituted with O, N or S, and may further may comprise saturated or unsaturated bonds.

As noted above, the term naphthenic acid may also used to identify *all* of the carboxylic acid content of a crude oil which can include alkyl substituted acyclics (including "fatty" acids), aromatic acids, carbazoles and isoprenoid acids. More complex acid structures with two, three and even four carboxylic groups (tetrameric acids) have been identified as well as structures containing heteroatoms (O, $O_4$, S, OS, $O_2S$, $O_3S$, N, NO, $NO_2$, $N_2O$) have been found in certain crude oils.

The specific acids may be tetrameric acids, in the molecular weight range of 1227 to 1235 Da. The acids homologous series corresponds to empirical formula of $C_{80}H_{138}O_8$, $C_{80}H_{140}O_8$, $C_{80}H_{142}O_8$, $C_{80}H_{144}O_8$ and $C_{80}H_{146}O_8$ with double bond equivalences (DBE) ranging from 12 to 8, indicating 8 to 4 rings in the hydrocarbon skeleton, respectively.

References to naphthenic acid include naphthenate and *vice versa* unless the context clearly specifies otherwise.

Hydrocarbon compositions, which can be analysed by a method of the invention, include crude oil, or partially purified crude oil, or an oil or substance obtained from crude oil following subsequent crude oil distillation, for example petroleum, kerosene, or paraffin. The method may be practised on samples obtained from crude oil directly, or from sludges, oil deposits, oil emulsions, or tars which have been prepared for sample analysis. The method covers such organic acids as can be extracted from "raw" petroleum crude oil liquid samples as received after extraction (either dry or water containing), as well as acids extracted from oilfield production system deposits, scales, sludges and emulsions, and including acids extracted from petroleum fractions of crude oils.

The crude oil may be a raw extract from a ground reservoir of oil following extraction, or it may be present in a refinery product stream, such as a distillate, fraction or other residue from a process unit. The hydrocarbon composition may also be dispersed in water. Methods of the invention are therefore applicable to the analysis of waste water from a refinery where the hydrocarbon composition is dispersed in the water.

Preparation for sample analysis may include appropriate steps to remove particulate and/or solid matter, excess water or other impurities. Excess water may be removed by a process of alternate heating and cooling of the sample, followed by centrifugation to remove the water. Alternatively, the water may be removed manually. The heating process may be carried out in an inert atmosphere, e.g. under nitrogen or helium or other inert gases.

The separation of naphthenic acid from the hydrocarbon composition may be achieved by means of a process such as dialysis. A dialysis procedure uses a membrane selected to be permeable to certain substances so as to permit a separation of the substance of interest from the remainder of the substances present. In the present case, the membrane may be permeable to low molecular weight naphthenic acids of molecular weight less than 600 daltons to diffuse across the dialysis membrane. A preferred dialysis procedure is the closed sack distillative reflux dialysis method which comprises extraction by dialysis under refluxing solvents. In such a method, the sample may be placed in a membrane dialysis sack (a.k.a. finger cot). Sack details are as follows. Supplier DEK Northern Europe, Granby Industrial Estate, Weymouth, Dorset DT4 9$^{TH}$, UK, Part no 173035. Sack/finger cot specifics are - Anti-Static latex ISO 5 (Class 100 7C), with the proprietary anti-static agent yielding average surface resistance of 5 x 10 12 Ohms Per square unit. Washed powder-free 3 ml latex cleanroom processed and packaged in ESD safe inner bags and covering the small. Medium, large and extra large sizes (Product Numbers PN-173033, 173034, 173035 and 173057). During the distillation phase of the procedure, the membrane sacks may suitably be placed into a holder or a basket.

At the conclusion of the dialysis procedure, the solvent dialysate surrounding the dialysis sack is removed for sample preparation prior to application to the macroreticular ion-exchange resin for analysis. The sample may be further concentrated, or otherwise prepared for application onto the resin. The sample may be dissolved and volumetrically prepared in organic solvents.

The dialysis residue containing the higher molecular weight naphthenic acid content is then removed (and which may be itself used for separate application to another macroreticular ion-exchange resin for analysis of the content of high molecular weight species in the sample).

Alternatively, the step (i) of separating naphthenic acid from the hydrocarbon composition and the step (ii) may be combined by direct addition of the hydrocarbon composition to the resin.

Suitable solvents may be a straight chain or branched C1-C10 alkane. The term $C_1$-$C_{10}$ includes the groups methyl, ethyl, propyl, butyl, pentyl, hexyl, heptyl, octyl, nonyl, and decyl. The alkane may be pentane, hexane, and or heptane. Preferably, hexane may be used as the dialysis extraction solvent.

Suitably, the resin is packed into the form of a column so as to assist the analysis of the sample. Where the resin is packed into the form of a column, the sample is loaded onto the column at one end.

The macroreticular ion-exchange resin may be a macroporous ion-exchange resin, suitably composed of a polymeric material. The polymeric material may be optionally cross-linked. For example, a suitable macro-reticular resin may be composed of cross-linked styrene divinylbenzene copolymers. A preferred type of such cross-linked styrene divinylbenzene copolymer macroreticular resins is Amberlyst 15® and/or Amberlyst A31 (Rohm & Haas).

The macroreticular resin is modified in that it comprises transition metal ions co-ordinated with an amine or ammonia in which the metal ion amine/ammonia co-ordinated complexes are bound to the surface of the resin. Suitably, the transition metal ion is supplied in the form of a water-soluble salt, which can be subjected to admixture with the amine

or ammonia to co-ordinate the transition metal ion. Suitable water-soluble salts include, but are not limited to, sulphate, nitrate, carbonate, halide (i.e. fluoride, chloride, bromide, iodide). The resultant complex can be formed in situ on the column where the amine or ammonia is already bound to the column.

The transition metal ion may be one or more the following: a Period 4 transition metal ion, a Period 5 transition metal ion, a Period 6 transition metal ion or a Period 7 transition metal ion. Alternatively, the transition metal ion may be a Group 3 (III B), Group 4 (IV B), Group 5 (V B), Group 6 (VI B), Group 7 (VII B), Group 8 (VIII B), Group 9 (VIII B), Group 10 (VIII B), Group 11 (I B), or Group 12 (II B) transition metal ion.

Period 4 transition metal ions include: Scandium (Sc), Titanium (Ti), Vanadium (V), Chromium (Cr), Manganese (Mn), Iron (Fe), Cobalt (Co), Nickel (Ni), Copper (Cu), Zinc (Zn). Period 5 transition metal ions include: Yttrium (Y), Zirconium (Zr), Niobium (Nb), Molydenum (Mo), Ruthenium (Ru), Rhodium (Rh), Palladium (Pd), Silver (Ag), Cadmium (Cd). Period 6 transition metal ions include: Lutetium (Lu), Hafnium (Hf), Tantalum (Ta), Tungsten (W), Rhenium (Re), Osmium (Os), Iridium (Ir), Platinum (Pt), Gold (Au).

Group 3 (III B) transition metal ions include Scandium (Sc), Yttrium (Y), Lutetium (Lu). Group 4 (IV B) transition metal ions include Titanium (Ti), Zirconium (Zr), Hafnium (Hf). Group 5 (V B) transition metal ions include Vanadium (V), Niobium (Nb), Tantalum (Ta). Group 6 (VI B) transition metal ions include Chromium (Cr), Molydenum (Mo), Tungsten (W). Group 7 (VII B) transition metal ions include Manganese (Mn), Rhenium (Re). Group 8 (VIII B) transition metal ions include Iron (Fe), Ruthenium (Ru), Osmium (Os). Group 9 (VIII B) transition metal ions include Cobalt (Co), Rhodium (Rh), Iridium (Ir). Group 10 (VIII B) transition metal ions include Nickel (Ni), Palladium (Pd), Platinum (Pt). Group 11 (I B) transition metal ions include Copper (Cu), Silver (Ag), Gold (Au). Group 12 (II B) transition metal ions include Zinc (Zn), Cadmium (Cd).

Preferred transition metal ions may include metal ions selected from the group consisting of Copper (Cu), Silver (Ag), Nickel (Ni), Cobalt (Co), Iron (Fe), Manganese (Mn), Molybdenum (Mo), Chromium (Cr), and Platinum (Pt). More particularly preferred transition metal ions may be Copper (Cu), Nickel (Ni) or Molybdenum (Mo).

In one embodiment of the invention, the transition metal ion is not added to the resin (or resin prepared in the form of a column) but rather the complex is prepared on the resin or resin column, so the reaction to form the complex takes place on the resin or resin column.

The transition metal ion is co-ordinated with an amine or ammonia. The transition metal ion is suitably adsorbed on to the column and the amine or ammonia is then suitably fixed on to the metal to form the appropriate metal/ammonium or metal/amine complex in situ. For example, where the metal is copper, the use of ammonia will lead to the formation of the corresponding cuprammonium complex and the use of an amine will lead to the formation of the appropriate cupramine complex.

The amine may be an amine of general formula:

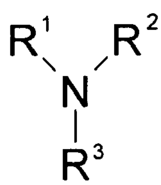
(I)

in which $R^1$, $R^2$ and $R^3$ may each independently be Hydrogen (H), or a $C_1$-$C_6$ straight chain or branched alkyl group, optionally substituted with Oxygen (O). The term $C_1$-$C_6$ includes the groups methyl, ethyl, propyl, butyl, pentyl, and hexyl.

Where one or more of $R^1$, $R^2$ and $R^3$ are an alkoxy group it may comprise from 1 to 14 alkoxylated groups, selected from ethylene oxide, propylene oxide or butylene oxide and mixtures thereof.

The amine may be a linear, branched chain or fused-ring system amine. For a fused-ring system amine, either $R^1$ and $R^2$, or $R^2$ and $R^3$ or $R^3$ and $R^1$ can be joined together to form a ring which may be saturated or unsaturated.

The amine may be a primary, a secondary or a tertiary amine. Suitable amines include methylamine ($CH_3NH_2$), ethylamine ($C_2H_5NH_2$), ethylene diamine ($NH_2CH_2CH_2NH_2$), pyridine, morpholine, cyclohexamine, or benzylamine. Where all groups R are Hydrogen, the general formula will represent ammonia.

A coordinated complex of the transition metal ion and the amine of general formula (I) or ammonia may be formed by in situ on the macro-reticular resin by adsorption entrapment. Macroreticular resins, e.g. in the form of a macroreticular resin column are specifically designed to trap metal ions.

After loading the sample onto the resin or resin column, it suitably allowed to equilibrate to permit the formation of naphthenic acid complexes with the bound transition metal ion amine/ammonia complex. After a sufficient period of time has elapsed, the resin or resin column is then washed to remove unbound sample.

The column may be washed with solvents such as xylene and/or heptane which may assist in removal of heavy

oil residues. Xylene and/or toluene may also be used for this purpose. A final wash of isopropanol may be used to swell the resin to assist in washing the column of excess residues.

Washing the resin or resin column may therefore comprise one or more of the following steps:

- wash with xylene / heptane mixture
- wash with xylene / toluene mixture
- wash with isopropanol/water

Suitable mixtures of the wash solvents may be as follows: heptane/ xylene in the ratio of from 0:90 to 100:10, suitably, of from 40:50 to 60:50, preferably of about 50:50.

Eluting the naphthenic acid-metal ion complexes may be achieved with a suitable eluant selected from the group consisting of $C_6$-aryl, straight chain or branched $C_1$-$C_6$ alkyl alcohol or $C_6$-aryl alcohol, ammonia, and straight-chain or branched $C_1$-$C_6$ alkylamine and mixtures thereof.

The term $C_1$-$C_{10}$ includes the groups methyl, ethyl, propyl, butyl, pentyl, hexyl, heptyl, octyl, nonyl, and decyl.

The $C_1$-$C_6$ alkylamine, includes primary, secondary and/or tertiary amines. The $C_1$-$C_6$ alkyl alcohol includes primary, secondary and/or tertiary alkyl alcohols. $C_6$-aryl alcohol includes phenol.

Preferably, the straight chain or branched $C_1$-$C_6$ alkyl alcohol may be methanol, ethanol, and/or isopropanol. Preferably, the $C_1$-$C_{10}$ alkane is *n*-heptane.

The straight chain or branched $C_1$-$C_6$ alkyl alcohol may be formulated as an aqueous solution or may be used. The straight chain or branched $C_1$-$C_6$ alkyl amine and/or ammonia may be formulated as an aqueous solution or as a solution of ammonia in a straight chain or branched $C_1$-$C_6$ alkyl alcohol as alcoholic ammonia or amine. For example, methanolic ammonia.

Suitably, the proportion of $C_1$-$C_6$ alkyl alcohol to ammonia or amine may be in the ratio of from 95:5 to 80:20.

Following, the elution of the complexed naphthenic acid-ammonia or amine co-ordinated transition metal ion, the eluate may be concentrated by evaporation of the elution solvents. The concentration of the same may be achieved by evaporation under an inert gas atmosphere (e.g. nitrogen or other inert gases like helium), lyophilisation or any other generally suitable means.

Quantitation of the concentration of naphthenic acid-metal ion complexes present in the eluate may be achieved by measuring the absorbance of complexed naphthenic acid-ammonia or amine co-ordinated transition metal ions present in the sample and correlating this absorbance from a calibration graph. The absorbance can be correlated to the concentration of the complexes present by using known standards as controls.

The formation of the complex yields an ion complex with a characteristic UltraViolet radiation (UV) absorption signal by virtue of the co-ordinated transition metal ion present in the complex. Quantitation of the amount of naphthenic acid in a sample may therefore be achieved using an assessment of the UV absorption of a sample by spectrophotometry techniques compared to known reference samples containing defined amounts of naphthenic acid, including samples where naphthenic acid is absent. Suitably, UV absorption measurement may be made at 215 to 225nm, preferably at 218 to 222nm.

Quantitation could also take place using means to detect the formation of radioactive complexes or complexes that give rise to fluorescent products.

The present invention therefore provides a newly developed screening method for isolation of and fingerprinting of a diagnostic family of low molecular weight naphthenic acids implicated in naphthenate soap material formation in oil production plants and in refinery facilities.

In a preferred embodiment of the invention, a method for the quantitation of naphthenic acid content in a hydrocarbon sample may comprise the steps of:

(1) Separating and purifying low molecular weight naphthenic acids from crude oil samples by e.g. dialysis

(i) using a dialysis membrane sack in the separation of the low molecular weight naphthenic acids fraction from raw crude oil samples, petroleum fractions and deposits containing naphthenate soap (solids, soap-emulsions and sludges); and

(ii) optionally using a metal mesh basket of specific dimensions may be used to protect the dialysis membrane sacks from bursting during distillative reflux dialysis extraction of test samples, with a basket holder for multiple samples to be run;

(2) Selecting a transition metal from the transition metal series;

(3) Preparing of a macro reticular resin in a transition metal form with a ligand as described above and forming resin

as a column;

(4) Applying the sample to the column;

(5) Washing the column, and subsequent washing to remove excess uncomplexed sample with a heptane/xylene wash, or mixtures thereof, Isopropanol-water mixtures; and finally eluting the complexed naphthenic acid-co-ordinated ammonia/amine transition metal ion with an alcoholic amine solution;

(6) Identifying the ultraviolet (UV) absorption lambda max wavelength of metal co-ordinated naphthenic acid complexes from samples under test, with reference to known copper naphthenates (pure standards) and actual field naphthenate deposits acid fraction extracts, converted to the metal co-ordinated ammonium / amine forms, by elution through the macroreticular resin column;

(7) Identifying the optimum and maximum absorbance values required for metal co-ordinated naphthenic acid complexes, from samples under test, such that the absorbance remains within an acceptable lambda max range for the calibration graph;

(8) Identifying that the dilution of high absorbances obtained from the metal co-ordinated naphthenic acid complexes meet with the maximum absorbance value recommended and that this dilution is linear;

(i) Establishing a calibration procedure using low molecular weight naphthenic acids extracted from actual samples of naphthenate soap deposits/sludges and crude oil emulsions recovered from actual oilfield production and refinery processes;

(9) Identifying the minimum volume of coloured, metal co-ordinated naphthenic acid complexes required for UV analysis and for UV fingerprint testing and identification of the minimum detectable naphthenic acids amount (Limit of Detection, LOD) in test solution, which is 0.25 mg in this method for LMWAs;

(10) Quantifying the amount of low molecular weight naphthenic acids (LMWA) obtained in the dialysate residue of test samples (e.g. crude oils, sludges) relative to known LMWA fractions extracted from actual oilfield deposit samples;

(11) Normalisation the %wt LMWA fraction in the dialysate residue of test samples back to the original crude oil; and

(12) Normalising the %wt LMWA fraction of stabilised crude oil samples with previously unknown %wt LMWA fraction / problematic soaps quality to the known problematic crude oils.

Step (1)(i) may comprise distilling off the dialysate solvent in order to achieve separation of low molecular weight naphthenic acids in the dialysate residue. In other embodiments, the sample may be added directly to the resin or resin prepared in the form of a column.

The UV fingerprinting method of crude oils allows mg content of LMWA to be determined from the calibration graph and this can then be expressed as the %wt LMWA fraction of each oil under test. This is used to screen oils by comparing them versus blank oils that do not form naphthenates in the oilfield and by comparing the results to crude oils known for having soap and emulsion problems during production, including oils that give calcium naphthenate deposition.

Preferably the sample for analysis may be a crude oil sample, for example impure crude oil from exploration/ appraisal wells, drill stem tests, test separator samples, downhole high pressure live samples (e.g. Schlumberger/Expro SPS/MDT/RCI type samples) or refinery charge to the heat exchangers. The sample could be crude oil residues recovered from oil refinery operations/plants. The sample could be crude oil or naphthenate deposits found in upstream petroleum processes or from refinery operations.

In the separation procedure, where dialysis is used, it may be preferable to use a basket or a holder for the dialysis membrane, such as a metal basket. The metal basket may be constructed of stainless steel which will not be oxidized or react with Low Molecular Weight Naphthenic (LMWA) acids.

Preferably, transition metal is chosen from a water soluble salt of Copper, Nickel or Molybdenum.

In the preparation of the co-ordinated transition metal ion, preferably the nitrogen containing ligand is chosen from ammonia, ethylamine and ethylene diamine.

After loading the column and washing of excess unbound transition metal ion and/or unreacted impurities from the sample, the elution solvent sequence can be chosen from a straight chain or branched $C_1$-$C_{10}$ alkane, $C_1$-$C_{10}$ alkane

- $C_6$ aromatic solvent mix, aqueous $C_1$-$C_6$ alcohol, neat $C_1$-$C_6$ alcohol, and $C_1$-$C_6$ alcoholic ammonia / $C_1$-$C_6$ amine.

The neat alcohol may be methanol. The alkane may be chosen from C5, C6 or C7 alkanes or mixtures thereof, e.g., pentane, hexane and/or heptane. In the alcohol/amine mixture, the alcohol may be ethanol, methanol or isopropanol or mixtures thereof, but preferably methanol.

In a laboratory scale version of the method, the resin may be prepared as a column, for example in a burette. The burette may have a physical resin bed dimension of 35 cm x 10mm, with reservoir height of 15 cm. The resin may be Amberlyst A15®. In such embodiments, the sample dialysis weight may range between 1 to 8 g and the dialysis time may range between four to twelve hours. The preferred sample weight may be 6 grams. The preferred dialysis time may suitably be 6 hours.

Where dialysis is used in the separation of the sample prior to loading onto the resin, the dialysis reflux-extraction solvent may be pentane, hexane or heptane with hexane being preferred.

In a laboratory scaled version of the method, the charge of the material from dialysate on the burette sized column may range from 75-250 mg, with a preferred range 75 - 150 mg. In such embodiments, the elution rate may range from 1.5 - 3 ml/min, with a preferred rate of 2 ml/min. In such embodiments, the volume of eluates to be used may be standardised at 100 ml volume. Glacial acetic acid or formic acid can be used in sample loadings to a maximum of 20 micro litres on the burette sized columns.

After elution of the naphthenic acid complexes from the column, the alcohol or alcohol-ammonia solutions may be gently evaporated off to dryness under a nitrogen blanket.

The residues obtained from the evaporation of the alcohol or alcoholic ammonia may be collected and combined in a suitable amount of alcoholic ammonia solution for UV analysis starting at 3 millilitres and concentrated by evaporation to a residue.

Standard naphthenic acids may be obtained as follows from source hydrocarbons. Crude naphthenate soaps may be isolated from crude oil by a clean up step then followed by a dialysis extraction procedure. Warm heptane and ambient temperature xylene may be used in the clean up step. After the clean-up step, the crude naphthenate soap may then be treated to remove oil and residues/asphaltenes. The dialysate residue (DSR) after dialysis extraction may be treated with warm (60°C) xylene-glacial acetic acid (1:1), extract solution, evaporated to dryness then re-dialysed twice to give the purified field naphthenic acids on evaporation to a residue.

The analysis of the amount of naphthenic acid in a sample or a standard may be carried out by measuring the UV absorption of a sample, wherein the lambda max UV absorption is set at 218-222 nm +/- 2 nm. Preferably, the maximum absorbance at lambda max range of 218-222nm is set at 2.5 +/- 0.5A. The LMWA absorbances across the UV range may be measured on a UV-computer link and results normalized to those of known crudes, enabling prediction of problematic soaps formation.

The oil analysis method describe in the present application involves a novel combination of analytical techniques, which concentrate up the high molecular weight acids fraction of any crude oil sample. While concentrating on actual crude oils samples with a view to using this analysis technique to screen them for their acid fraction contents, it is equally possible to use this method on samples of sludge, solid deposits, treatment chemicals, drilling muds, distilled crude oil fractions, oil products and so on. These isolated acids with other high molecular weight components are then complexed on to a specific type of resin column for later elution and ultraviolet (UV) spectrophotometric analysis. Contaminants are washed off the column first, leaving behind the organic acids of interest. Results show a gradation of % complexed acids versus good and bad oils relative to probability of soap formation resulting in oil production and/or oil refining problems.

This technique holds great potential as an oil screening tool for new oilfields and even can be used on current production operations for troubleshooting present problems, i.e. with compartmentalised oil reservoirs, on a well-by-well basis, you can determine the wells that give fluids that will generate soaps and those that do not, allowing a map to be built up of fluids produced versus potential soap generation. The method procedure is relatively rapid, simple to learn and run, very accurate when compared to the other oil screening techniques currently available and only a small quantity of the test oil sample is required for this test method.

Using such a method to rank oils with respect to their risk of causing soap problems, in upstream oilfield production systems and in downstream crude refining plants, is the basis of this patent application.

Methods in accordance with the present invention will enable the determination of the % weight low molecular weight naphthenic (%LMW) acids fraction of crude oils, leading to a classification fingerprint index. This index will assist in evaluating crude oils from new oilfield developments because once analysed, they can be classified with respect to their degree of risk for naphthenate soap/emulsion formation and corrosion tendencies, relative to the % LMW naphthenic acids. This prediction of problematic naphthenate soap/emulsion formation is becoming critically important for new oilfield developments and the design and operation of production facilities, such as refineries. Operating companies need to know up front if a new oil will be a naphthenate soap former or not, as the associated flow assurance issues can be very severe and will heavily influence the facilities design [5]. In addition, such operating companies can look at their overall oilfield development plan and decide upon the various alternative measures they could take in controlling any naphthenate related production problems, from wells to the topsides export oil system. These options would include dilution of problem

oils with oils that will not form naphthenates, dilution of the produced waters to negate naphthenate formation, injection of treatment chemicals for naphthenate soap control, employing up front high pressure separators and so on [2 to 5 & 8]. This oil screening tool for naphthenates potential will have a big impact on the decisions made by various oilfield operating companies in the future.

The present invention has taken the analysis of heavy naphthenic acid soap precursors a step further by using a combination of exhaustive distillative-reflux and dialysis separation techniques, giving a concentration of high molecular weight naphthenic acids and then further reaction of the naphthenic acids by trans-metallation to metal ligand co-ordinated-ammonium naphthenates, on a macroreticular resin column. This leads to highly UV active transition metal-complexed compounds, which can be detected, with a suitable scanning UV spectrophotometer, down to the 1.25 mg level, in actual test solutions.

The bulk concentration of polar compounds based upon molecular weight can be performed by a separation technique known as dialysis, which is here paired with a second separation phase using macroreticular resin. Normal column chromatography, using alumina and silica gel, separates materials by polarity strength, which is related to molecular weight. Separation by normal column work is not bulk separation in groups but fine separation due to material polarities. Preparatory Thin layer Chromatography (PTLC) gives the same types of separations at narrow MW ranges, but it is difficult to use TLC on crude oil samples, as alumina will react with the naphthenic acids.

Dialysis is normally used in the well-known medical fields in blood purification re kidney dialysis. References are available in the food industry for pumped dialysis in non-organic electrolytes [27]. This type of dialysis is not the closed sack distillative reflux dialysis but pumped dialysis or direct current dialysis at ambient conditions.

References also exist for the macroreticular resins but mainly used in the environmental field. Silver is chemically and physically bonded to a macroporous resin (macroreticular), to enable it to scavenge out biocides from effluent waters [28]. No reference has been found, though, for the use of these macroreticular resins in their amino/ammonia-metal ligand form, in separation of naphthenic acids from crude oils, by column complexation and trans metallation. Types of anion exchange resins have been used to separate naphthenic acids [10, 29] but not in the area of direct metal ammonia-coordinated complexations of naphthenic acids from crude oils. This is one reason why this methodology for patent exists because it has not been tried before.

Based on the synopsis of the naphthenate soap problems discussed previously there is a need for a less expensive, less time-consuming, more accurate and commercially more cost-effective analytical technique for screening oil samples for naphthenate formation potential. This development work here has shown advances in the application of combined analytical techniques, including separation of a specific molecular weight range of organic acids from crude oil and complexation of that acid fraction, to give a method, which can be used in actual onsite oilfield laboratories, as well as in a central land-based laboratory.

The methods provide means for quantifying the unknown % LMWA in the dialysate residue, calculating the % non-HMWAs in the DR of refinery streams thereby giving a cleanliness index, determining inherent and potential fouling indices, quantifying the amount of naphthenic acids in desalter disposal water streams for environmental concerns, and/or determining of the emulsion tendency of the crude oil at the desalter stage compared to a known emulsified crude oil.

The quantitative methods of the present invention enables the isolation of low molecular weight and high molecular weight naphthenic acids to such a degree that their concentration can be determined in units of milligrams (mg) acid per litre of oil (mg/l) and the results can be expressed as % weight (%wt) acid fraction of oil, henceforth known as % High/Low Molecular Weight Acid (%HMWA/LMWA) fraction. This specific group of LMW naphthenic acids that this method targets are known to be in part responsible for the formation of problematic calcium naphthenate soap solids deposition and/or in the formation of sodium or potassium soap emulsion problems in many oilfield production facilities and refinery systems worldwide.

The present invention therefore also provides methods applicable to crude oils, deposits, process disposal water, refinery product streams and bottoms asphalts to determine the ratio of high molecular weight to the low molecular weight naphthenic acids.

Preferred features for the second and subsequent aspects of the invention are as for the first aspect *mutatis mutandis.*

The invention will now be further described by way of reference to the following Examples and Figures which are provided for the purposes of illustration only and are not to be construed as being limiting on the invention. Reference is made to a number of Figures in which:

FIGURE 1 shows a graphical representation of the content of Table 2.

FIGURE 2 shows a graphical representation of the content of Table 3.

FIGURE 3 shows a graphical representation of the content of Table 4.

FIGURE 4 shows a graphical representation of the content of Table 5.

FIGURE 5 shows a graphical representation of the content of Table 6.

FIGURE 6 shows a graphical representation of the content of Table 7.

FIGURE 7 shows a graphical representation of the content of Table 8.

FIGURE 8 shows a graphical representation of the content of Table 9.

FIGURE 9 shows a graphical representation of the content of Table 10.

FIGURE 10 shows a graphical representation of the content of Table 11.

FIGURE 11 shows a graphical representation of the content of Table 12.

Definitions

HMWA - High molecular weight naphthenic acids (molecular weight greater than 600); LMWA - Low molecular weight naphthenic acids (molecular weight less than 600); HAC - High Acid Crude feedstocks such as West African, SJV and Venezuelean Crudes; BR - Non-distillable residue; DSR - Dialysate Residue.

Methods

The naphthenate soap solids from the West African field were purified to remove asphaltenes, crude oil, resins, mineral matter and particulates to yield a cleaner HMW soap. The HMW acids were liberated and mass spectroscopy, Thin Layer Chromatography (TLC) and Infra Red Spectroscopy (IR) performed on these. The MS spectra matched Oil Plus, Norwegian and French research results indicating we have collected the 600 -1230 HMWAs for calibration. TLC indicated that naphthenic acids were present similar to another set of HMWAs liberated from a North Sea naphthenate deposit.

An elution sequence was designed to wash off resins, oil and asphaltenes from the column. A metal co-ordinated complex is formed which is soluble in methanolic ammonia solution. Resins, tars and bitumens are not soluble in aqueous Methanol-Methanolic ammonia solutions but in xylene, which is used in the clean up procedure on the column.

All subsequent results were judged against a calibration graph constructed with these purified HMWAs. The metal coordination complexes of the naphthenic acids were acidified in acetic acid / xylene and TLC and mass spectroscopy were performed on the resulting solution to show that the Naphthenic acids were being complexed on a column.

TLC in an acidic mobile phase to break the complex to acids and on the acetic acid / xylene solution showed naphthenic acid spots compared to developed colours of Fluka naphthenic acids. These were compared to the acids from the parent material and to another North Sea naphthenic acid liberated from the respective solids from that field.

Low molecular weight acids were collected from the dialysate residue (DSR). A normal TAN titration and TLC were performed. Then the LMWAs were complexed on the column to construct a calibration graph.

All samples are cleaned up, dialysed and treated on the column. The UV spectrum is compared to the standard HMWA and LMWA graphs. A calculation is performed to relate back to the original sample. The HMWAs results are entered into our NPI calculation to give an index. This is correlated to known Naphthenate fouling or non-fouling database of NPI.

The emulsion tendency can be similarly ranked versus the worst emulsion forming crude oil with the highest LMWAs known in practice.

Analyses indicate that naphthenic acids are distributed in the dialysis residues and the dialysate residues (DSR). The entire DR does not consist solely of naphthenic acids. Correcting for the naphthenic acids content then the Non-Acid content can be calculated. The latter has been found to have a wide range from 30-90 % of the DRs. These non-acidic materials could be other HMW materials, insoluble naphthenate soaps and hot hexane insoluble materials. Asphaltenes, resins, polymers, gums and mineral matter are included here.

Thus analysis of the DR gives the HMW Naphthenic acids which are corrosive in heat exchangers and capable of forming fouling soaps or acting as binders for the non acid constituents. The DSR gives the LMW Naphthenic acids with potential for being distilled into the fractions causing emulsions, failure of fractions to pass quality control procedures

## Example 1: Analysis of crude oil for naphthenic acid content

Initially, the crude oils under test were stabilised gravimetrically utilising nitrogen-purged heating to remove light ends <70°C and the water content would be separated by the standard heated centrifuge method.

A stainless steel metal cage was constructed to hold the dialysis sack containing the sample and locked in position such that the sack does not expand to bursting. The dimensions of the metal cage were chosen such that several metal cages containing samples can be stacked to fit the 1 litre dialysis reservoir. This then meant that approximately eight oil samples could be dialysed in one distillative reflux dialysis experiment. Samples were initially stabilized by gently heating up a known weight of sample under a nitrogen blanket to prevent oxidation. Each sample undergoes a heat - cool procedure to constant weight. The weight loss of volatiles is recorded and used in a correction procedure. The free water is removed by pipette or by a 60 seconds centrifuge spin (heated). The amount of removed free water (weight loss) is also used in a correction procedure when required.

The stabilised oils were then extracted by use an exhaustive distillative-reflux membrane dialysis apparatus and the sack retained non-dialyzable high molecular weight acids (HMWA), with a MW range of approximately 600 amu and above (including the naphthenate precursor ARN-type acids), which included any naphthenate soaps present. These would then be gravimetrically collected by removal of the extraction solvent by heating at 70 deg C, under a Nitrogen blanket. (DR weight is recorded and converted to % DR), minus the sack weight), and termed as the dialysis residue (DR). The samples include solids, soap-emulsions and sludges.

The solvent dialysate (DS) containing low molecular weight compounds including low molecular weight (LMW) naphthenic acids was then subject to further analysis and the dialysis residue comprising the high molecular weigh (HMW) was not used further.

The DSR's were volumetrically prepared in normal organic solvents and eluted through a specially prepared copper ammonium macroreticular resin column using a series of chosen solvents (by experiments). This combination of solvents removes, residual oil and asphaltenes/resins/waxes, leaving the low molecular weight naphthenic acids of interest, complexed (bound) on the column.

Copper is chosen here in the example but transition metals give coloured complexes from their oxidized states when they accept a ligand. Copper, Nickel and Molybdenum are safe to work with. Chromium is toxic so not used here. The transition metal is chemically bonded on to the macroreticular resin and then prepared to accept an amine/ammonium ligand. The macroreticular resin column is first converted to the transition metal form and co-ordinated with ammonia or amines from above. The ammonia/amines attach to the transition metal and rapidly impart a characteristic blue colour to the co-ordinated metal still attached to the resin. This complex now reacts with naphthenic acids to give coloured complexes based on the amount of naphthenic acids attached to the metal. For example the cuprammonium ion does not give a UV spectra, but once complexed with naphthenic acids, a UV absorption is observed in the copper -carbonyl UV absorption area.

Xylene and heptane are used to clean up heavy oil residues if these are present in the DSR charged to the column. Xylene and toluene were found to be good solvents for these residues. IPA-water solvent clean up after the xylene wash, affords a swelling of the column to expand the pores of the resin to remove the residual xylene and thus the residual heavy residues if present. The sequential solvent washing was identified after lengthy trials and it removes contaminants while leaving the acids of interest on the column. These coordinated metal complexed naphthenic acids would then be collected in two experimentally chosen elution solvents and carefully evaporated under nitrogen purge, slowly to dryness. The residues would then be combined, re-dissolved in an ammoniacal solution for spectrophotometric analysis.

Method of sample preparation

As above, the Dialysis Residue is collected and the hexane solvent is evaporated off and the % Dialysis Reside (%DR) is determined gravimetrically.

The dialysate comprises the hexane solubles and contains relatively lower molecular weight materials from generally 600 MW and decreasing. The HMW materials and hexane insolubles are retained as the DR in the Dialysis membrane sack.

The Dialysate solution was set up in a distillation flask with thermometer and a Nitrogen sparge, 1 bubble /second, and the hexane solvent was carefully distilled off at around 70°C to leave approximately 50 m L solution of Dialysate Residue (DSR) in hexane.

The DSR solution was transferred to a 100 m L volumetric flask and volumetrically made up to 100 m L with hexane. This is the DSR stock solution. The weight in grams of DSR in the stock solution was calculated from the formula:

$$((100 - \%DR) \times \text{Weight grams of sample taken for dialysis})/100$$

The Column Method:

Based on the column dimensions described herein, sufficient aliquot of the DSR stock solution was taken to give approximately 500 mgs of DSR and this was transferred to a measuring cylinder.

The column was prepared ready for accepting sample as described herein. The aliquot of DSR solution is transferred to the column and the method followed as described herein.

The complexes of LMWAs formed were collected in solution form as per the normal method and the dried residues were redissolved combined in methanolic ammonia for UV spectrophotometric analyses.

Preparation of the DSR of the Standard

A crude oil with known high emulsion characteristics, was prepared and dialysed as above. The dialysate solution was collected as above and a stock solution of the DSR were made up in hexane.

Preparation of the Amberlyst 15 Column to collect the Naphthenic Acids

An Amberlyst A 15 macroreticular resin column was prepared according to the following steps:

the preparatory column was set up using column dimensions 30cm (1 foot) resin bed x 2.5cm (linch) ID with a solvent reservoir head of 15cm (6 inches)
the resin was washed with 100 mL of 5% Hydrochloric acid solution
the resin was washed with approximately 100 mL distilled water, until the pH of the eluate was around pH 7
100 mL 5% Sodium Hydroxide solution was added to the column and eluted at 1 mL / minute
the column was washed with water until pH was near pH 7
the column was washed with approximately 100 mL methanol
the column was washed with 100 mL xylene
the column was washed with 100 mL hexane - xylene solution 80-20 ratio

Collection of LMW Naphthenic Acids for Calibration:

an aliquot was taken of the DSR of the standard crude to give 2.5 grams of DSR
this aliquot was added to the preparatory column
the hexane solvent was eluted at 1 m L/minute, until the eluates were near colourless
the column was eluted with hexane-xylene 80-20 mixture until eluates were colourless
the column was eluted with 5% solution of Acetic acid in Methanol until eluate was colourless
approximately 150 m L Acetic acid-methanol solution were required
the methanolic-acetic acid solution was evaporated off under Nitrogen until the vapours were neutral to litmus paper
the drying was finished off in an oven at 55 deg C.
then cooled and dissolved in xylene, filtered through a Whatman No 1 filter paper to remove sodium acetate
the filtrate of the Naphthenic acids was collected in a weighed beaker the evaporation of solvent was repeated and oven finished.
the weight of Naphthenic acids obtained was calculated and the filtrate was dissolved in 100 m L hexane-xylene 80-20 mix solvent to give a stock LMW Naphthenic acid solution.

Checking integrity of the LMW Acids:

a TLC method was performed compared to Fluka and Aldrich Commercial naphthenic acids
to identify that Naphthenic acids are present a standard ASTM TAN D664 assay method was performed
an acid value test was used to detect naphthenic acids in the stock solution

Preparation of Calibration Graph:

an aliquot of stock LMW Naphthenic acid solution was taken and the complexation on the Cu MRR resin column was performed as per standard method.
the standard method was followed with different aliquots of Naphthenic acid solution
the UV absorbance was read at 218 nm +/- 2 nm
a calibration graph was constructed

Interpolation of Sample UV absorbance to mg LMW Naphthenic acids

from the gradient of the graph, one Absorbance unit at 218nm was found to be equivalent to 0.6 mg of LMW Naphthenic acids in final test solution

Example 2: Determination of the ratio of high molecular weight to the low molecular weight naphthenic acids in crude oil samples

The example shows the evaluation of the fouling tendencies of two crude oils and the product streams from the pipestill.

The following samples were analysed: (i) Crude Oil, (ii) Crude Oil to Desalter (PS02), (iii) Raw Jet Fuel, (iv) Diesel Feed to E120, (v) VGO, VAC SS2 to CFHU, (vi) LGVO, VAC SIDE, STREAM 1 to FCCU, (vii) HAGO, (viii) Residual, vacuum pipe still bottoms-pitch

The samples were subjected to four types of analyses in this part of the project for fouling and corrosion interpretations. The original samples were analysed for TAN values using the normal ASTM D664 technique, then the Bottoms Residue (BR) was separated from the Dialysate Residue (DSR) and both fractions analysed for high and low molecular weight Naphthenic acids using a patent pending technique. The original samples were also analysed for the total acids.

All results and observations are tabulated in the Results section. The data from the tables are graphically presented with interpretations of results alongside the graph. A discussion of the methodology is presented in the Discussion section.

Results

The results with interpretations and implications from the data obtained are as summarised follows:

Table 1

| | Tests on Original Sample | | | BR-N | DSR-N | DSR-N | BR | BR | BR | Original |
|---|---|---|---|---|---|---|---|---|---|---|
| Sample | TAN D664 | %BR | % DSR | % HMWA | % LMWA | TAN D664 | % Acids | % Non-Acids | ppm non-acids Normalized | NFI (or NPI) |
| TK1704 | 3.11 | 12.1 | 88.9 | 3.91 | 2.8 | 2.06 | 32.2 | 67.8 | 82K | 12.7 |
| PS02 | 1.55 | 10.7 | 90.3 | 1.98 | 7 | 1.56 | 18.6 | 81.4 | 87K | 6.4 |
| Jet Fuel | 0.24 | 0.32 | 99.7 | 0.15 | 0.1 | 0.29 | 47.1 | 52.9 | 1693 | 3.2 |
| Diesel | 1.14 | 0.94 | 99.1 | 0.05 | 0.4 | 1.95 | 5.2 | 94.8 | 8911 | 0.9 |
| HAGO | 2.17 | 0.74 | 99.3 | 0.1 | 2.4 | 2.35 | 30.6 | 69.4 | 5136 | 1.2 |
| LVGO | 2.39 | 0.47 | 99.6 | 0.27 | 1.1 | 2.71 | 57.6 | 42.4 | 1993 | 7.8 |
| VGO | 2.68 | 5.63 | 94.3 | 5.62 | 2.6 | 1.69 | 36.3 | 63.7 | 36K | 6.6 |

(continued)

| | Tests on Original Sample | | | BR-N | DSR-N | DSR-N | BR | BR | BR | Original |
|---|---|---|---|---|---|---|---|---|---|---|
| Sample | TAN D664 | %BR | % DSR | % HMWA | % LMWA | TAN D664 | % Acids | % Non-Acids | ppm non-acids Normalized | NFI (or NPI) |
| Residual | 5.7 | 36.4 | 63.7 | 9.69 | 0.4 | 0.55 | 26.5 | 73.4 | 26K | 30.2 |
| BR= Bottoms Residue LMWA=Low Molecular Weight Acids<br>DSR= Dialysate Residue -N= Normalized Data to original<br>HMWA=High Molecular Weight Acids Mod= Modified<br>NFI= Naphthenate Fouling Index (equivalent to NPI) | | | | | | | | | | |

The details of the results are shown in the following tables:

Table 2

| | Naphthenate Fouling Index (NFI) | |
|---|---|---|
| Ranking No | Sample | NFI |
| 1 | Residual | 30 |
| 2 | TK1704 | 12.7 |
| 3 | LVGO | 8 |
| 4 | VGO | 7 |
| 5 | PS02 | 6 |
| 6 | Jet fuel | 3 |
| 7 | PS19 HAGO | 1 |
| 8 | Diesel | 1 |
| NOTE: All Fouling Interpretations are based on High Molecular Weight Naphthenic Acids Only | | |

The Residual should be a known fouling fraction. All others compare against it. PS02 crude is 2x lower fouling than TK1704 Crude. LVGO and VGO are fouling fractions and relatively high. Fouling indices compared to a Naphthenic acid crude oil. PS 19 HAGO & Diesel are relatively low fouling. An NFI fouling factor of 7 is considered a high NFI. The HMWA's will act as binders to the non-HMWA's forming tight deposits in the HTEXs. A NFI value of 2 or below is considered to indicate a low probability for the occurrence of a fouling issue. The probability of a fouling issue to occur will increase with the increasing NFI.

The content of Table 2 is represented graphically in Figure 1.

The NFI increases in the correct order depending on the % HMWA's determined as set out in Table 3 below. TK 1704 crude has 2x the fouling tendency compared to PSO2 crude. The jet fuel has a moderate fouling tendency in heat exchangers.

Table 3

| NAP Acid Distribution vs. TAN | | | | |
|---|---|---|---|---|
| | NORMAL TAN | %BR | %HMWA's | %LMWA's |
| CRUDE TK1704 | 3.11 | 12.10 | 3.91 | 2.8 |
| CRUDE PS02 | 1.56 | 10.70 | 1.98 | 7.0 |
| Jet Fuel (O) | 0.24 | 0.32 | 0.15 | 0.1 |
| DIESEL (O) | 1.12 | 0.94 | 0.05 | 0.4 |
| PS 19-HAGO(O) | 2.17 | 0.74 | 0.1 | 2.4 |
| LVGO (O) | 2.39 | 0.47 | 0.27 | 1.1 |
| VGO(O) | 2.68 | 5.63 | 5.62 | 2.6 |

(continued)

| NAP Acid Distribution vs. TAN | | | | |
|---|---|---|---|---|
| | NORMAL TAN | %BR | %HMWA's | %LMWA's |
| Residual (O) | 5.70 | 36 | 9.69 | 0.4 |

Table 3 is a collection of important data points showing positive gradations in TAN's and %HMWA's as fractions come off at increasing temperatures. Similar series for BR's & %LMWA's in atmospheric section and another positive series for the vacuum section.

Table 4

| | BOTTOMS RESIDUES |
|---|---|
| | % Bottoms Residue (BR) |
| CRUDE TK 1704 | 12.10 |
| CRUDE PS02 | 10.70 |
| Jet Fuel (O) | 0.32 |
| DIESEL (O) | 0.94 |
| PS19-HAGO(O) | 0.74 |
| LVGO (O) | 0.47 |
| VGO | 5.63 |
| Residual (O) | 36 |

Residual % BR (36%) not included in graph so that the graph can now show comparisons of relatively lower % BR values. Results show the two crude oils have similar % BRs. The BRs will consist of general hexane insoluble materials and compounds > 600 MW. HMWA's, Napthenate soaps, asphaltenes, Resins, gums and particulate matter are expected in the BRs. The VGO has a high BR value and increases the probability of finding HMWA's.

Table 5

| | HMWA's v LMWA's | |
|---|---|---|
| | %HMWA's | %LMWA's |
| CRUDE TK1704 | 3.91 | 2.8 |
| CRUDE PS02 | 1.98 | 7.0 |
| Jet Fuel (O) | 0.15 | 0.1 |
| DIESEL (O) | 0.05 | 0.4 |
| PS19-HAGO(O) | 0.1 | 2.4 |
| LVGO (O) | 0.27 | 1.1 |
| VGO | 5.62 | 2.6 |
| Residual (O) | 9.69 | 0.3 |
| (O) = normalized vs. the original sample | | |

The two crude oils are different in % HMWA's & % LMWA's. The %HMWA's increase as temperatures increase showing a high rate of increase to VGO (2.6%) & Resid fractions (9%). The %LMWA's also increase in that order in the atmospheric section but a second series increase to over 2.5% in the VGO. As expected the residual contains only 0.4% LMWA's but 9% HMWA's. As theorized, the higher the % LMWA's the lower the TAN compared to reverse observed for HMWA's.

Table 6

| NORMAL TAN ASTM D664 VARIATION | |
|---|---|
| | NORMAL TAN |
| CRUDE TK 1704 | 3.11 |
| CRUDE PS02 | 1.56 |
| Jet Fuel (O) | 0.24 |
| DIESEL (O) | 1.12 |
| PS19-HAGO(O) | 2.17 |
| LVGO (O) | 2.39 |
| VGO | 2.68 |
| Residual (O) | 5.70 |
| (O) = normalized vs. the original sample | |

The two crude oils show different TANs by the normal method. TANs increase as expected as temperatures increase in the Pipestill. As LMWA's decrease as temperatures increase down the column then the increases in TANs are due to the HMWA's- implicated in corrosion and fouling tendencies.

Table 7

| | %BR Product Streams |
|---|---|
| CRUDE TK1704 | 12.10 |
| CRUDE PS02 | 10.70 |
| Jet Fuel (O) | 0.32 |
| DIESEL (O) | 0.94 |
| PS19-HAGO(O) | 0.74 |
| LVGO (O) | 0.47 |
| VGO | 5.63 |
| Residual (O) | 36.00 |
| (O) = normalized vs. the original sample | |

% BRs increase 3 fold from the Jet Fuel to the Diesel Fraction in the Atmospheric section but increases rapidly in the vacuum section which is an expected result. Residual % BR (36%) is not plotted here to afford depth and clarity to the graph as 36% would swamp the y-axis.

Table 8

| | DSR TAN to Original |
|---|---|
| CRUDE TK1704 | 2.06 |
| CRUDE PS02 | 1.55 |
| Jet Fuel (O) | 0.29 |
| DIESEL (O) | 1.95 |
| PS19-HAGO(O) | 2.35 |
| LVGO (O) | 2.71 |
| VGO | 1.69 |

(continued)

| | DSR TAN to Original |
|---|---|
| Residual (O) | 0.55 |
| (O) = normalized vs. the original sample | |

There is a high contribution to the overall TANs of the fractions from the LMWA's as the % DSRs on Atmospheric fractions and LVGO are in the 99% DSRs area. This decreased for the Vacuum section of the Pipestill for VGO and Residual as LMWA's in these fractions are decreased. This is to be expected and it appears that HMWA's breaking up in the Vacuum Residual section does occur here, providing LMWA's to the VGO stream.

Table 9

| Evaluating the BRs - % acid foulants | | | |
|---|---|---|---|
| Sample | %HMWA's in BR | % BR | %Non-HMWA's in BR |
| CRUDE TK1704 | 32.2 | 12.10 | 67.8 |
| CRUDE PS02 | 18.6 | 10.70 | 81.4 |
| Jet Fuel (O) | 47.1 | 0.32 | 52.9 |
| DIESEL (O) | 5.2 | 0.94 | 94.8 |
| PS19-HAGO (O) | 30.6 | 0.74 | 69.4 |
| LVGO(O) | 57.6 | 0.47 | 42.4 |
| VGO | 36.3 | 5.63 | 63.7 |
| Residual (O) | 26.5 | 36.00 | 73.5 |
| (O) = normalized vs. the original sample | | | |

PS02 crude has a lower (19%) % HMWA fouling material in BR compared to TK 1704 Crude (32%) in the BRs of these crudes. The BRs are essentially high molecular weight compounds and/or hexane insoluble Naphthenic acid materials. All fractions show significant concentrations of non-HMWA's (40-90%) in the HMW BRs. These are most likely the soaps of Nap acids and/or materials physically transported up the column by foaming or velocity of fluids. HMWA's in VGO and Residual fluids will cause more fouling and could break up to LMWA's in follow on processes leading to corrosion predictions.

Table 10

| Non-Acidic components in the BRs | | | |
|---|---|---|---|
| Sample | %Non-HMWA's in BR | %BR | ppm potential foulants |
| CRUDE TK1704 | 81.4 | 12.10 | 98494 |
| CRUDE PS02 | 67.8 | 10.70 | 72546 |
| Jet Fuel (O) | 52.9 | 0.32 | 1693 |
| DIESEL (O) | 94.8 | 0.94 | 8911 |
| PS19-HAGO(O) | 69.4 | 0.74 | 5136 |
| LVGO (O) | 42.4 | 0.47 | 1993 |
| VGO | 63.7 | 5.63 | 35863 |
| Residual (O) | 73.5 | 36.00 | 264600 |
| (O) = normalized vs. the original sample | | | |

All fractions show significant concentrations of non-HMWA's (40-90%) in the HMW BRs. These are most likely the soaps of Nap acids and/or materials physically transported up the column by foaming, surfactant effects or velocity

of fluids. These can include gums, polymers, particulate matter etc. In VGO, more likely to be dispersed resins, asphaltenes and tars

Table 11

| Sample | BR results ppm potential non-HMWA's foulants |
|---|---|
| CRUDE TK1704 | 98551 |
| CRUDE PS02 | 72521 |
| Jet Fuel (O) | 1693 |
| DIESEL (O) | 8915 |
| PS19-HAGO(O) | 5139 |
| LVGO (O) | 1993 |
| VGO | 35863 |
| Residual (O) | 264745 |
| | |
| Sample | ppm potential non-HMWA's foulants |
| Jet Fuel (O) | 1693 |
| DIESEL (O) | 8915 |
| PS19-HAGO (O) | 5139 |
| LVGO (O) | 1993 |
| VGO | 35863 |

The non-HMWA's foulants decrease from the Diesel fraction to the LVGO but a very high concentration of extraneous foulant(s) is observed in the VGO, correlates with the BR having a high value at 5-6 %. Dispersed resins, tars and asphaltenes could be cause of high values here.

Table 12

| | ppm HMWA's foulants |
|---|---|
| Jet Fuel (O) | 1500 |
| DIESEL (O) | 500 |
| PS 19-HAGO(O) | 1000 |
| LVGO (O) | 2700 |

The VGO is not included, as its ppm is very high 35K ppm. The ppm HMWA's foulants increase as temperatures increase in the Pipestill from diesel to LVGO and further on.

Discussion

All samples were cleaned up, separated and analyzed. The analysis results are compared to the standard HMWA and LMWA graphs. A calculation is performed to relate back to the original sample. The HMWA's results are entered into the NFI calculation to give an index. This is correlated to known Naphthenate fouling or non-fouling database of NFI.

The emulsion tendency can be similarly ranked versus the worst emulsion forming crude oil with the highest LMWA's known in practice.

Analyses indicate that Naphthenic acids are in the Bottoms residues and the Dialysate Residues. The entire BR does not consist solely of Naphthenic acids. Correcting for the Naphthenic acids content then the Non-Acid content can be calculated. The latter ranged 40-90 % of the BRs. As indicated in the Conclusion section, these non-acids could be other HMW materials and hot hexane insoluble materials. Asphaltenes, resins and mineral matter are included here.

Thus analysis of the BR gives the HMW Naphthenic acids which are corrosive in heat exchangers and capable of forming fouling soaps or acting as binders for the non-acid constituents. The DSR gives the LMW Naphthenic acids with potential for being distilled into the fractions causing emulsions failure of fractions to pass quality control procedures.

Observations

(a) TANs in relationship to HMWA's and LMWA's

The normal TAN method shows a general gradation in total Naphthenic acid properties of the crude oils and product streams (ref page 13). It is noted that the percent high molecular weight acids are low in relative terms from the Jet Fuel, Diesel, LVGO and HAGO fractions at < 0.5% HMWA's

The TANs of the Jet Fuel to HAGO fractions are contributed mainly by the LMWA's contents 0.1 and 2.5% LMWA's respectively. VGO and Residual have the highest % HMWA's and highest TAN's. The two crude oils, TK 1704 and PS02 are different in TANs (3 and 1.6 TAN respectively) and different in percent HMWA's content (4% and 2% approx) and the two crude oils however also contrast each other in their % LMWA's content (3% and 7% LMWA's respectively).

(b) Naphthenate Fouling Index NFI

The calculated Naphthenate Fouling Index (NFI) based on a data base of fouling & non-fouling crudes, high and low TANs, indicates that the residual has the highest fouling index (31) and there is a decreasing gradation from the residual to the jet fuel. However the NFI indicates LVGO and VGO to be high (8 and 7 respectively) fouling whilst jet fuel has an average medium fouling character. The two crude oils PS02 and TK 1704 are again different in the NFI (6 and 12 NFI respectively). The HAGO and Diesel fractions are of low probability of fouling

(c) The low molecular weight materials in the distillable residues (DSR) - relationship to overall tan contribution

The DSRs of the fractions contribute their TANs to the overall TAN values. This shows that the LMWA's are relatively medium-high in TAN values depending on the % DSRs and % LMWA's. in these fractions. The VGO has a high LMWA's (2.6%) content and thus a reasonable high contribution of TAN (1.7 TAN) to the original sample.

It is suggested that the HMWA's in the residual at 9-10 % HMWA's are being fractured in the vacuum section to give the appropriate LMWA's of the correct boiling range to be distilled off with the VGO fraction. The two crude oils, TK1704 and PS02, have different TANs (2.31 - 1.76) in their dialysate residues (DSRs) suggesting different % of molecular weight ranges of LMWA's.

(d) The High Molecular Weight / hexane insoluble materials in the bottoms residues (BRS

The bottoms residues contain the high molecular weight acids, which are implicated in fouling and corrosion mechanisms. Results indicate that the crude oils, TK 1704 and PS02 have similar concentrations of these bottoms residues (12% and 11% respectively). The bottoms residues gradually decrease in concentration from the Diesel to HAGO to LVGO fractions (0.9%, 0.7% and 0.5 % respectively). The Jet Fuel has a 0.3% hexane insolubles in the BR which is considered high for a jet fuel. The high molecular weight material in the BRs increase in the VGO and Residual samples (6% and 36% respectively)

(e) The High Molecular Weight Acids in the BRS

The crude oils, TK 1704 and PS02, although having comparable bottoms residue concentrations (12% and 11% respectively), the % HMWA's vary 32% to 18% respectively, our technique overcomes the weakness of the pentane insolubles which will not detect this diagnostic feature to fouling and corrosion potentials. All BRs of the fractions, except Diesel (5% HMWA's in BR) have high concentrations of HMWA's.

(f) Non-High Molecular Weight Acid foulants in the BRS

The non-HMWA's vary 40 to 90%. Both of these results indicate not only a high potential to fouling as per the NFI above for Naphthenic acids, but also inherent fouling potentials from the non-HMWA's and general corrosive characteristics.

The concentrations of non-HMWA's gradually decrease in values from the Diesel fraction to the LVGO (10 K ppm to 2K ppm respectively) but increases very sharply for the VGO fraction (35K ppm), Fouling by insoluble, high

molecular weight materials classed as non-HMWA's, is implicated for all fractions. In the BR of VGO, it is suggested that these materials are highly dispersed insoluble particulates of resins, tars, asphalts, mineral matter etc. For the residual the non-acid materials in this fraction are the insoluble high molecular weight asphalts, bitumens, mineral matter, ash etc

(g) Implications of the new test in evaluating the residual bottom of the barrel experiment

The new analytical procedure tells us that the residual contains valuable materials (64%), which are soluble in hot hexane. The hot hexane does not extract high molecular weight acids and the acid value of the distillable residue is 0.55 TAN, a significant decrease from nearly 6 TAN value.

The LMWA's in the residual is only 0.4% and thus the major contributor to the overall acid contents is the high molecular weight acid, which is not soluble in hot aliphatic solvents. Evaluating this data, a process of hot solvent extraction can yield a workable feedstock leaving 30-40% for asphalts.

Conclusions

The standard pentane insoluble test which would give the similar values as our hexane insoluble test and would not characterise that the two crude oils, TK 1704 and PS02, are dissimilar in respect if their problematic naphthenic acid contents

The results indicate that PS 1704 has twice the high molecular weight acid (HMWA) concentration in the BR, than PS02.

This fact indicates that TK1704 will possess higher fouling and corrosion tendencies than PS02 crude oil.

In contrast the PS02 oil has 2.5x more low molecular weight acids (LMWA's) than TK 1704 which definitely predicts that PS02 will cause more emulsion issues in desalters, poor desalting water quality, corrosion in units and naphthenic acids in product distillates.

The proprietary Naphthenate Fouling Index (NFI) calculation is an index developed to predict the probability of a fouling issue to occur due to the deposition of naphthenate soaps. The NFI calculation predicts a gradation of Indices from the worst fouling residual fraction to the least expected fouling, the diesel fraction. The NFI for the two crude oils, TK1704 and PS02, predicts TK1704 has twice the probability of fouling HMWA's and soaps than the PS02 crude. (Refer Table 1)

The method detects that fractions from the Jet Fuel to HAGO contain hundreds of ppm of HMWA's and extraneous insoluble non-acid materials. These materials should not be found in these fractions.

The test results can be diagnostic in troubleshooting refinery process problems with respect to fouling, corrosion versus physical conditions in a unit. The above tells us that a physical carry over is implicated in the movement of HMWA and insoluble non-acid materials up the column.

The results prove by logical extension to refinery technology that LMWA's should not be found in the residual fraction. Only 0.3% LMWA's is present in the residual but the VGO contains high % LMWA's. High Molecular Weight Acids are being fractured in the residual to produce LMWA's for the VGO stream.

The presence of relatively high % HMWA's in the VGO and residual means that we can interpret that these two fractions possess qualities for high fouling and corrosion in integrated refinery units and ancillary equipment handling and accepting the fractions.

The method suggests that in a bottom of the barrel experiment, hot aliphatic solvent extraction of the residual have 64% of organic materials leaving 36% insoluble residuum containing asphaltenes, resins, high molecular weight acids/soaps and mineral matter.

The pipestill side-cuts have all shown an amount of Naphthenic Acid contamination. The effects of these acids on the unit that receives these products needs to be considered. For example, the LVGO feedstock to the FCCU contains 0.3%HMWA and 1.1%LMWA. These Naphthenic acids and their soaps will have implications at the heat exchangers, catalyst beds and slurry oil separation tanks.

References

1. Goldzal et al 3rd Intl Conf on Petroleum Phase Behaviour and Fouling, (2002), New Orleans, USA
2. Rousseau et al "Calcium Carbonate and Naphthenate Mixed Scale in Deep Offshore Fields", SPE 68307 (2001).
3. Vinstad et al "Fighting Naphthenate Deposition at the Heidrun Field", (2003). SPE Oilfield Scale Symposium, SPE 80375
4. Roden, J, "Calcium Naphthenate Control on the Blake Field", proceedings from Flow Assurance Europe (IQPC

18th - 19th November 2003, London) (2003).
5. M.S Turner & P.C Smith (2005), Controls On Soap Scale Formation, Including Naphthenate Soaps - Drivers And Mitigation, SPE Oilfield Scale Symposium, SPE 94339, Aberdeen, UK
6. Sorbie et al 'Naphthenate Formation In Oil Production: General Theories And Field Observations', RSC; Chemistry in the Oil Industry IX, Manchester, UK, 31 October - 2 November 2005
7. Baugh et al SPE 93011, SPE Intl Symposium on Oilfield Scale, Aberdeen, UK, 1-12 May 2005
8. Gallup, D. L.: "Ca-Naphthenates and Metallic Soap Deposits: Formation and Mitigation", IQPC - Flow Assurance - A Holistic Approach, December 2004, Kuala Lumpur (2004).
9. Haynes, D. (2002) Opportunities in Processing High TAN Crude. Presented at Hydrocarbon Asia (September 2002).
10. Medias et al, 5th SPE Oilfield Scale Symposium, SPE 80404, (2003) Aberdeen, UK.
11. Acevedo et al Energy Fuels 1999, 13, 333-335.
12. Lochte, H.L., & Littman, E.R., The Petroleum Acids and Bases., Chemical Publishing Co., Inc, NY 1955
13. Seifert, W.K & Howells, W.G. Anal Chem. 1969, 41, 554-568
14. Seifert, W.K & Teeter, R.M,. Anal Chem. 1969, 41, 786-795
15. Seifert, W. K, & Teeter R.M. Anal Chem. 1970, 42, 180-189
16. Oil Plus internal fluid characterisation reports (not published).
17. Naphthenic Acid Corrosion in Crude Distillation Units, Material Performance, 28, 1988, 32-43
18. Goldzal et al, SPE 74661, SPE Intl Symposium on Oilfield Scale, Aberdeen, UK, 2002
19. Brandl, O, PhD Thesis, Norwegian Inst of Sci and Tech, May 2005
20. Derungs, W.A. Corrosion, 1956, 12, 41-46
21. Babian-Kibala et al Amer. Chem. Soc., 1998,3, 106-110
22. Roussis et al (ExxonMobil) US20020086434 - July 4, 2002
23. Green et al High Resoluti. Chromatgr. 1994, 17, 427-438
24. Havre et al J Dispersion Sci. Techol. , 2002.
25. McCarthy, R. & Duthie, A.J. J. Lipid. Res. 1962, 3, 117-119
26. Havre, E. T, Colloid Polym. Sci. (2004), 282, 270-279
27. Membrane Dialysis: determination of sugars in Dairy Products, Metrohm UK, Laboratory Talk, 2 July 2004
28. Costin, C. R., Rohm & Haas Company, PA, US 4076622, 1975
29. Tomczyk et al Prepr.-Am, Chem, Soc., Div, Pet, Chem., 1998, 3, 125, 123-125
30. Brient J.A. Prepr. -J.Amer. Chem. Soc., Div. Pet. Chem. 1998, 3, 131-133

## Claims

**1.** A method for the quantitation of the naphthenic acid content of molecular weight less than 600 daltons in a hydrocarbon composition, comprising

(i) separating naphthenic acids of molecular weight less than 600 daltons from the hydrocarbon composition;
(ii) applying the separated naphthenic acids of molecular weight of less than 600 daltons to a macroreticular ion-exchange resin additionally comprising transition metal ions co-ordinated with an amine or ammonia in which the metal ion amine/ammonia co-ordinated complexes are bound to the surface of the resin;
(iii) washing the resin;
(iv) eluting the naphthenic acid-metal ion complexes from the resin; and
(v) quantitating the number of naphthenic acid-metal ion complexes present in the eluate from the resin.

**2.** A method as claimed in claim 1, in which the hydrocarbon composition comprises crude oil.

**3.** A method as claimed in claim 1 or claim 2, in which the step (i) of separating naphthenic acid from the hydrocarbon composition is carried out by dialysis.

**4.** A method as claimed in claim 1 or claim 2, in which the step (i) of separating naphthenic acid from the hydrocarbon composition and the step (ii) are combined by direct addition of the hydrocarbon composition to the resin.

**5.** A method as claimed in any one of claims 1 to 4, in which the macro reticular ion-exchange resin comprises a transition metal ion selected from Copper (Cu), Silver (Ag), Nickel (Ni), Cobalt (Co), Iron (Fe), Manganese (Mn), Chromium (Cr), and Platinum (Pt).

**6.** A method as claimed in any one of claims 1 to 5, in which the amine is of general formula:

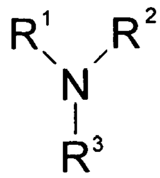

(I)

in which $R^1$, $R^2$ and $R^3$ may each independently be Hydrogen (H), or a $C_1$-$C_6$ straight chain or branched alkyl group.

**7.** A method as claimed in claim 6, in which the amine is ethyl amine, ethylene diamine, morpholine, pyridine, cyclohexylamine, or benzylamine

**8.** A method as claimed in any preceding claim, in which washing of the resin in step (iii) is carried out using xylene, toluene and/or heptane

**9.** A method as claimed in claim 8, in which the step (iii) is followed by a further wash using isopropanol-water mixture.

**10.** A method as claimed in preceding claim, in which the eluting the naphthenic acid-metal ion complexes in step (iv) is carried out using an eluant selected from the group consisting of straight-chain or branched $C_1$-$C_{10}$ alkane, $C_6$-aryl, straight chain or branched $C_1$-$C_6$ alkyl alcohol or $C_6$-aryl alcohol, ammonia, and straight-chain or branched $C_1$-$C_6$ alkylamine.

**11.** A method as claimed in claim 10, in which the straight-chain or branched $C_1$-$C_6$ alkyl alcohol is methanol, ethanol, and/or isopropanol.

**12.** A method as claimed in claim 10, in which $C_1$-$C_{10}$ alkane is *n*-heptane.

**13.** A method as claimed in claim 10 or 11, in which the straight chain or branched $C_1$-$C_6$ alkyl alcohol is an aqueous solution

**14.** A method as claimed in claim 10, in which the straight chain or branched $C_1$-$C_6$ alkyl alcohol is an anhydrous preparation.

**15.** A method as claimed in claim 10, in which the straight chain or branched $C_1$-$C_6$ alkyl amine and/or ammonia is an aqueous solution

**16.** A method as claimed in claim 10, in which the straight chain or branched $C_1$-$C_6$ alkyl amine and/or ammonia is formulated as a solution in a straight chain or branched $C_1$-$C_6$ alkyl alcohol.

**17.** A method as claimed in claim 16, in which the solution is methanolic ammonia.

**18.** A method as claimed in any preceding claim, in which the quantitating the number of naphthenic acid-metal ion complexes present in the eluate is carried out by measuring the UV absorption of the complexes.

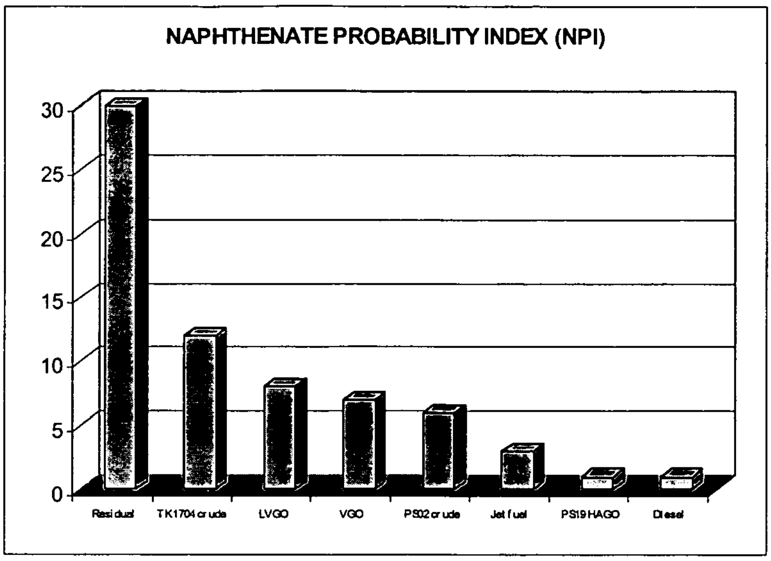


Figure 1

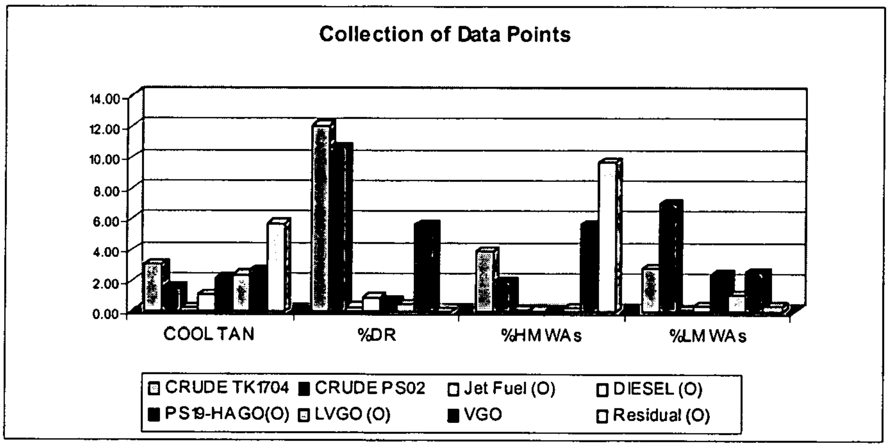


Figure 2

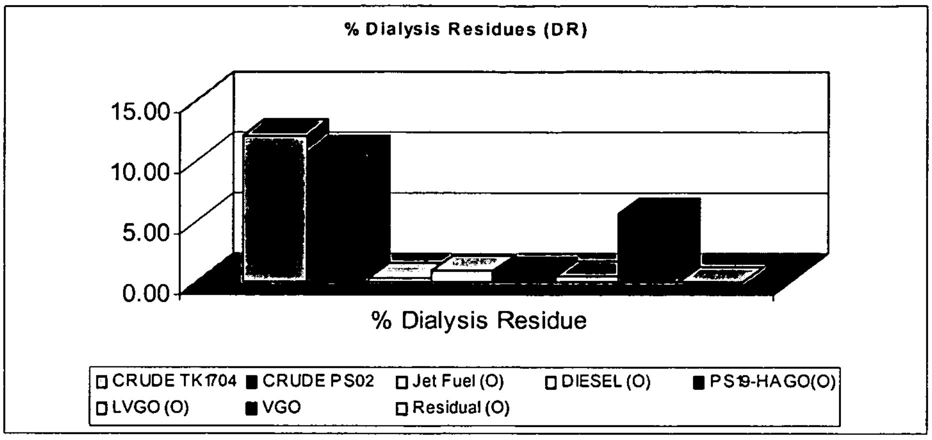


Figure 3

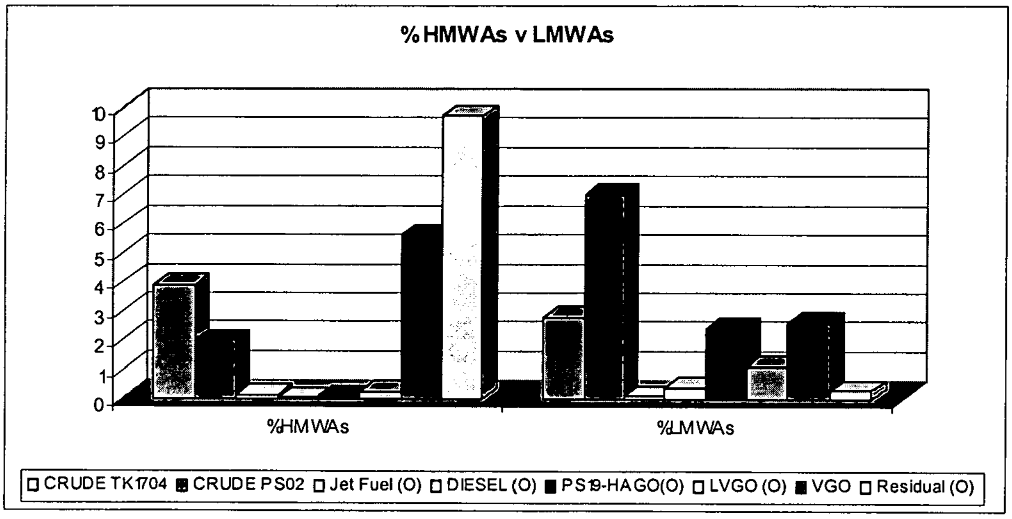


Figure 4

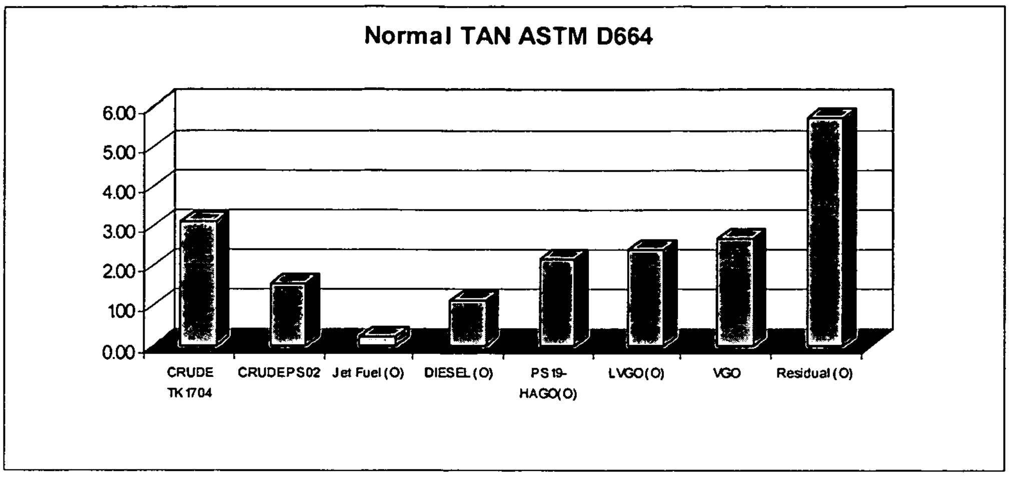


Figure 5

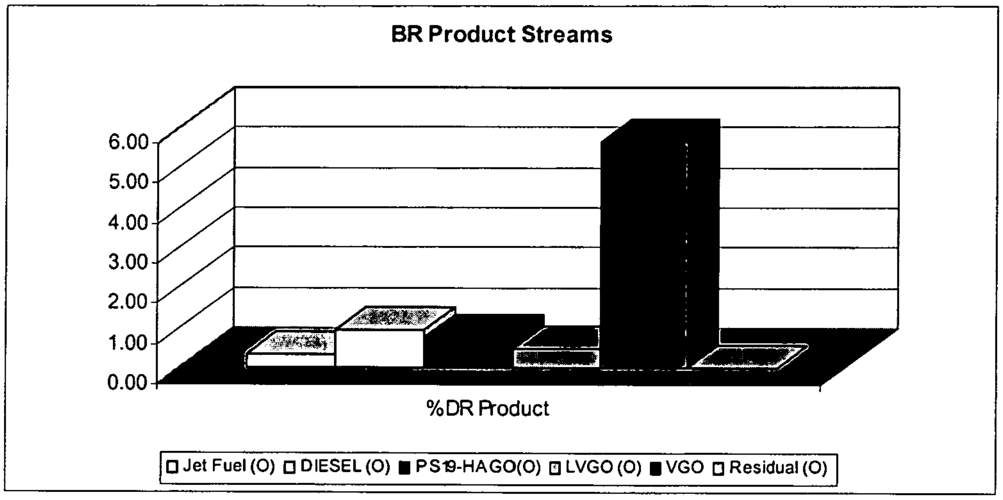


Figure 6

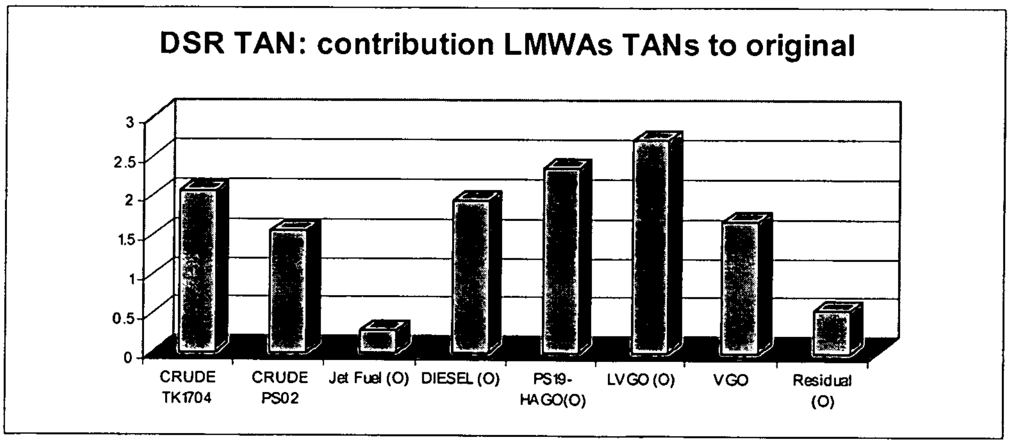


Figure 7

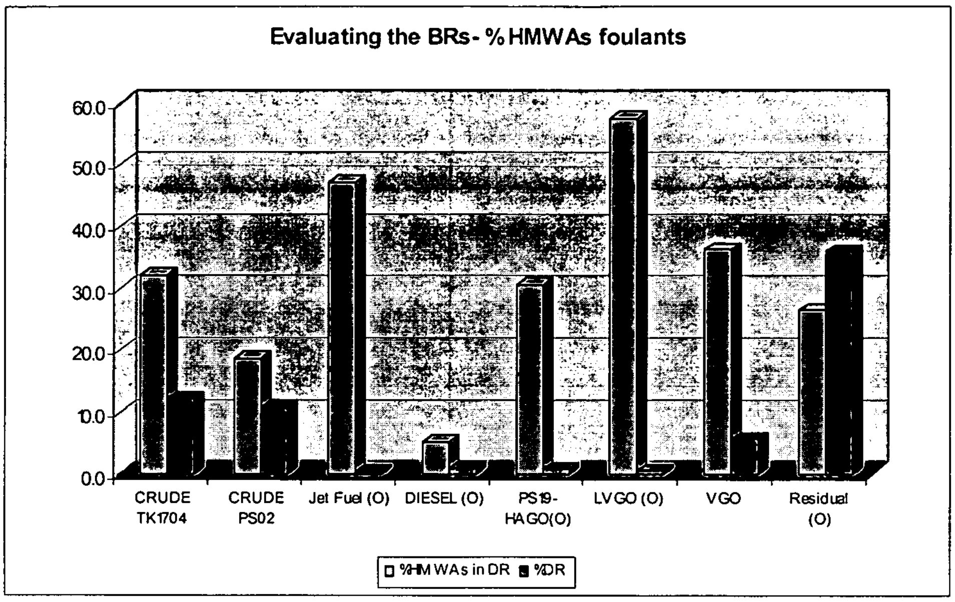


Figure 8

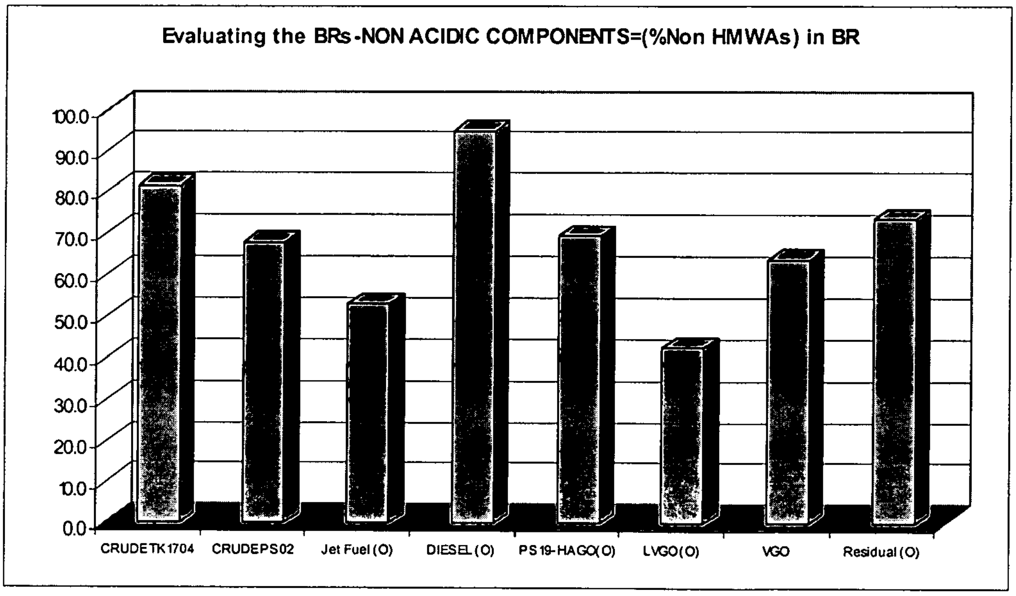


Figure 9

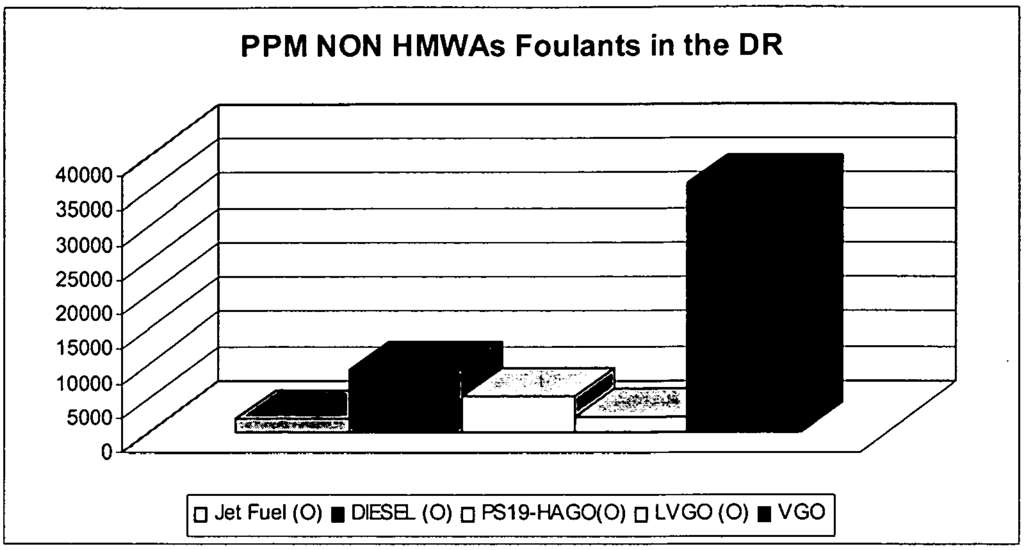


Figure 10

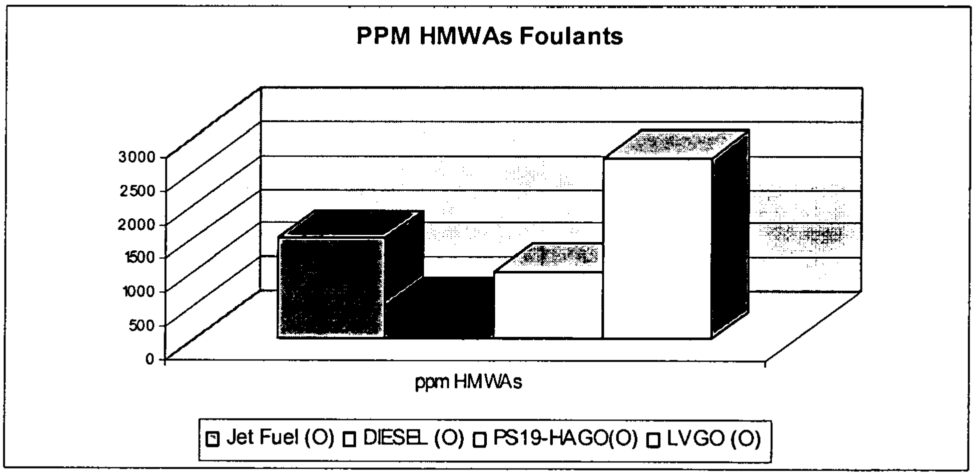


Figure 11

European Patent Office

# EUROPEAN SEARCH REPORT

Application Number
EP 07 25 2518

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | FAN TSENG-PU: "Characterization of naphthenic acids in petroleum by fast atom bombardment mass spectrometry" ENERGY & FUELS, vol. 5, no. 3, 1991, pages 371-375, XP002453023 AMERICAN CHEMICAL SOCIETY * abstract * * page 371, line 2 - page 372, line 23 * * figure 1 * * figure 5 * | 1 | INV. G01N30/06 B01D15/38 B01J31/16 G01N33/28 |
| A | JEWELL D M; WEBER J H; BUNGER J W; PLANCHER H; LATHAM D R: "Ion-Exchange, Coordination, and Adsorption Chromatographic Separation of Heavy-End Petroleum Distillates" ANALYTICAL CHEMISTRY, vol. 44, no. 8, July 1972 (1972-07), pages 1391-1395, XP002453024 AMERICAN CHEMICAL SOCIETY * abstract * * page 1392, column 2, paragraph 3 * * page 1393, column 1, line 11 - line 25 * * figure 1 * | 1 | TECHNICAL FIELDS SEARCHED (IPC): B01D G01N C10L C10M C22B B01J |
| A | WEBSTER P V; WILSON J N; FRANKS M C: "Macroreticular ion-exchange resins: some analytical applications to petroleum products" ANALYTICA CHIMICA ACTA, vol. 38, 1967, pages 193-200, XP002453020 ELSEVIER * abstract * * page 198, line 4 - page 200, line 5 * * tables V-VI * | 1 | |
| | -/-- | | |

The present search report has been drawn up for all claims

| Place of search | Date of completion of the search | Examiner |
|---|---|---|
| Munich | 28 September 2007 | Bravin, Michel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

4
EPO FORM 1503 03.82 (P04C01)

European Patent Office

# EUROPEAN SEARCH REPORT

Application Number
EP 07 25 2518

| Category | Citation of document with indication, where appropriate, of relevant passages | Relevant to claim | CLASSIFICATION OF THE APPLICATION (IPC) |
|---|---|---|---|
| A | HIRSCH R F; GANCHER R E; RUSSO F R: "Macroreticular chelating ion-exchangers" TALANTA, vol. 17, no. 6, June 1970 (1970-06), pages 483-489, XP002453021 PERGAMON * abstract * * page 484, line 5 - line 13 * * page 483, line 1 - line 4 * * figure 1 * | 1 | |
| A | SAAB J.; MOKBEL I.; RAZZOUK A C; AINOUS N; ZYDOWICZ N; JOSE J: "Quantitative Extraction Procedure of Naphthenic Acids Contained in Crude Oils. Characterization with Different Spectroscopic Methods" ENERGY & FUELS, vol. 19, no. 2, 2005, pages 525-531, XP002453022 AMERICAN CHEMICAL SOCIETY * abstract * * page 526, column 1, line 21 - page 528, column 1, line 9 * * figures 1,2 * | 1 | TECHNICAL FIELDS SEARCHED (IPC) |
| A | JONES D M; WATSON J S; MEREDITH W; CHEN M; BENNETT B: "Determination of Naphthenic Acids in Crude Oils Using Nonaqueous Ion Exchange Solid-Phase Extraction" ANALYTICAL CHEMISTRY, vol. 73, no. 3, 2001, pages 703-707, XP002453019 AMERICAN CHEMICAL SOCIETY * the whole document * | 1 | |

-/--

The present search report has been drawn up for all claims

| Place of search | Date of completion of the search | Examiner |
|---|---|---|
| Munich | 28 September 2007 | Bravin, Michel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

4

European Patent Office

# EUROPEAN SEARCH REPORT

Application Number
EP 07 25 2518

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GAIKAR V G ET AL: "Adsorptive recovery of naphthenic acids using ion-exchange resins" REACTIVE & FUNCTIONAL POLYMERS, ELSEVIER SCIENCE PUBLISHERS BV, NL, vol. 31, no. 2, September 1996 (1996-09), pages 155-164, XP004052642 ISSN: 1381-5148 * abstract * * table 1 * ----- | 1 | |
| A | RUDZINSKI W M; AMINABHAVI T M; SASSMAN S; WATKINS L M: "Isolation and Characterization of the Saturate and Aromatic Fractions of a Maya Crude Oil" ENERGY & FUELS, vol. 14, no. 4, 17 June 2000 (2000-06-17), pages 839-844, XP002453025 AMERICAN CHEMICAL SOCIETY * abstract * * table 3 * ----- | 1 | TECHNICAL FIELDS SEARCHED (IPC) |
| A | VADIM A. DAVANKOV AND ANDREW V. SEMECHKIN: "Ligand-exchange chromatography" JOURNAL OF CHROMATOGRAPHY A, vol. 141, no. 3, 21 December 1977 (1977-12-21), pages 313-353, XP002453026 ELSEVIER * page 319, paragraph B - page 321, paragraph E * * page 335, paragraph C - page 336, paragraph D * ----- | 1 | |

The present search report has been drawn up for all claims

| Place of search | Date of completion of the search | Examiner |
|---|---|---|
| Munich | 28 September 2007 | Bravin, Michel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

4

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- GB 0520847 A
- US 20020086434 A, Roussis et al (ExxonMobil)
- US 4076622 A, Costin, C. R., Rohm & Haas Company, PA


### Non-patent literature cited in the description


- **GOLDZAL et al.** *3rd Intl Conf on Petroleum Phase Behaviour and Fouling,* 2002
- **ROUSSEAU et al.** Calcium Carbonate and Naphthenate Mixed Scale in Deep Offshore Fields. *SPE 68307,* 2001
- **VINSTAD et al.** Fighting Naphthenate Deposition at the Heidrun Field. *SPE Oilfield Scale Symposium, SPE 80375,* 2003
- **RODEN, J.** Calcium Naphthenate Control on the Blake Field. *Flow Assurance Europe,* 18 November 2003
- **M.S TURNER ; P.C SMITH.** Controls On Soap Scale Formation. *Including Naphthenate Soaps - Drivers And Mitigation, SPE Oilfield Scale Symposium, SPE 94339, Aberdeen, UK,* 2005
- **SORBIE et al.** Naphthenate Formation In Oil Production: General Theories And Field Observations. *RSC; Chemistry in the Oil Industry IX, Manchester, UK,* 31 October 2005
- **BAUGH et al.** *SPE 93011, SPE Intl Symposium on Oilfield Scale, Aberdeen, UK,* 01 May 2005
- **GALLUP, D. L.** Ca-Naphthenates and Metallic Soap Deposits: Formation and Mitigation. *IQPC - Flow Assurance - A Holistic Approach,* December 2004
- **HAYNES, D.** Opportunities in Processing High TAN Crude. *Presented at Hydrocarbon Asia,* September 2002
- **MEDIAS et al.** 5th SPE Oilfield Scale Symposium. *SPE 80404,* 2003
- **ACEVEDO et al.** *Energy Fuels,* 1999, vol. 13, 333-335
- **LOCHTE, H.L. ; LITTMAN, E.R.** The Petroleum Acids and Bases. Chemical Publishing Co., Inc, 1955
- **SEIFERT, W.K ; HOWELLS, W.G.** *Anal Chem.,* 1969, vol. 41, 554-568
- **SEIFERT, W.K ; TEETER, R.M.** *Anal Chem.,* 1969, vol. 41, 786-795
- **SEIFERT, W. K ; TEETER R.M.** *Anal Chem.,* 1970, vol. 42, 180-189
- *Oil Plus internal fluid characterisation reports*
- Naphthenic Acid Corrosion in Crude Distillation Units. *Material Performance,* 1988, vol. 28, 32-43
- **GOLDZAL et al.** *SPE 74661, SPE Intl Symposium on Oilfield Scale, Aberdeen, UK,* 2002
- **BRANDL, O ; PHD THESIS.** *Norwegian Inst of Sci and Tech,* May 2005
- **DERUNGS, W.A.** *Corrosion,* 1956, vol. 12, 41-46
- **BABIAN-KIBALA et al.** *Amer. Chem. Soc.,* 1998, vol. 3, 106-110
- **GREEN et al.** *High Resoluti. Chromatgr.,* 1994, vol. 17, 427-438
- **HAVRE et al.** *J Dispersion Sci. Techol.,* 2002
- **MCCARTHY, R. ; DUTHIE, A.J.** *J. Lipid. Res.,* 1962, vol. 3, 117-119
- **HAVRE, E. T.** *Colloid Polym. Sci.,* 2004, vol. 282, 270-279
- Membrane Dialysis: determination of sugars in Dairy Products. *Metrohm UK, Laboratory Talk,* 02 July 2004
- **TOMCZYK et al.** *Prepr.-Am, Chem, Soc., Div, Pet, Chem.,* 1998, vol. 3 (125), 123-125
- **BRIENT J.A.** *Prepr. -J.Amer. Chem. Soc., Div. Pet. Chem.,* 1998, vol. 3, 131-133